(19) **Europäisches Patentamt**
**European Patent Office**
**Office européen des brevets**

(11) **EP 3 093 662 B1**

(12) **EUROPEAN PATENT SPECIFICATION**

(45) Date of publication and mention of the grant of the patent:
**15.05.2019 Bulletin 2019/20**

(51) Int Cl.:
*G01N 33/50* (2006.01)   *A61K 31/4164* (2006.01)
*A61P 35/02* (2006.01)   *G01N 27/62* (2006.01)
*G01N 30/26* (2006.01)   *G01N 30/72* (2006.01)
*G01N 30/88* (2006.01)   *G01N 33/68* (2006.01)
*G01N 33/574* (2006.01)   *G01N 33/49* (2006.01)
*G01N 33/53* (2006.01)

(21) Application number: **15735185.9**

(22) Date of filing: **09.01.2015**

(86) International application number:
**PCT/JP2015/050478**

(87) International publication number:
**WO 2015/105174 (16.07.2015 Gazette 2015/28)**

(54) **5-HYDROXY-1H-IMIDAZOLE-4-CARBOXAMIDE EFFECTIVE-DOSE/SENSITIVITY PREDICTION METHOD AND PREDICTION DEVICE, XANTHOSINE-MONOPHOSPHATE-AMOUNT MEASUREMENT METHOD, AND MYELODYSPLASTIC-SYNDROME TREATMENT AGENT AND TREATMENT METHOD**

VORHERSAGEVERFAHREN UND VORHERSAGEVORRICHTUNG FÜR WIRKSAME DOSIS/SENSITIVITÄT VON 5-HYDROXY-1H-IMIDAZOL-4-CARBOXAMID, XANTHOSIN-MONOPHOSPHAT-MENGENMESSVERFAHREN UND BEHANDLUNGSMITTEL UND BEHANDLUNGSVERFAHREN FÜR MYELODYSPLASTISCHES SYNDROM

PROCÉDÉ DE PRÉDICTION ET DISPOSITIF DE PRÉDICTION DE DOSE EFFICACE/SENSIBILITÉ DE 5-HYDROXY-1H-IMIDAZOLE-4-CARBOXAMIDE, PROCÉDÉ DE MESURE DE QUANTITÉ DE MONOPHOSPHATE DE XANTHOSINE, ET AGENT DE TRAITEMENT ET PROCÉDÉ DE TRAITEMENT DE SYNDROME MYÉLODYSPLASIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.01.2014   JP 2014003611**

(43) Date of publication of application:
**16.11.2016   Bulletin 2016/46**

(73) Proprietor: **FUJIFILM CORPORATION**
**Minato-ku**
**Tokyo 106-8620 (JP)**

(72) Inventors:
• **MURASE, Motohiko**
  **Ashigarakami-gun**
  **Kanagawa 258-8577 (JP)**
• **MATSUSHITA, Minako**
  **Ashigarakami-gun**
  **Kanagawa 258-8577 (JP)**
• **KOMATSU, Kensuke**
  **Ashigarakami-gun**
  **Kanagawa 258-8577 (JP)**
• **WATANABE, Kumiko**
  **Ashigarakami-gun**
  **Kanagawa 258-8577 (JP)**
• **KUWATA, Yusuke**
  **Ashigarakami-gun**
  **Kanagawa 258-8577 (JP)**
• **TAKEO, Atsushi**
  **Ashigarakami-gun**
  **Kanagawa 258-8577 (JP)**
• **YAMASHITA, Ryo**
  **Ashigarakami-gun**
  **Kanagawa 258-8577 (JP)**

(74) Representative: **Hoffmann Eitle**
**Patent- und Rechtsanwälte PartmbB**
**Arabellastraße 30**
**81925 München (DE)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 093 662 B1

(56) References cited:
WO-A1-2008/053530    WO-A1-2009/123297
JP-A- H1 151 885      JP-A- 2012 127 771
US-A- 4 753 935

- ISABELLE LAVERDIÈRE ET AL: "Liquid Chromatography-Coupled Tandem Mass Spectrometry Based Assay to Evaluate Inosine-5'-monophosphate Dehydrogenase Activity in Peripheral Blood Mononuclear Cells from Stem Cell Transplant Recipients", ANALYTICAL CHEMISTRY, vol. 84, no. 1, 3 January 2012 (2012-01-03), pages 216-223, XP055295182, US ISSN: 0003-2700, DOI: 10.1021/ac202404y
- Ferdi Sombogaard: "pharmacodynamic monitoring of IMPDH: a basis for optimized and individualized mycophenolate mofetil therapy", , 1 January 2010 (2010-01-01), pages 1-224, XP055321035, Retrieved from the Internet: URL:http://repub.eur.nl/pub/18478/100312_S ombogaard, Ferdi.pdf [retrieved on 2016-11-18]
- KIYOJI KIMURA ET AL.: 'Zoketsuki Shuyo ni Taisuru SM-108(4-Carbamoylimidazolium-5-olate) no Phase II study' JAPANESE JOURNAL OF CANCER AND CHEMOTHERAPY vol. 16, no. 1, 1989, pages 123 - 130, XP008182427
- DAISUKE TESHIMA: 'TDM for Second-Line Medicine (Mycophenolate Mofetil) in the Immunosuppresive Medication' JAPANESE JOURNAL OF THERAPEUTIC DRUG MONITORING vol. 23, no. 2, 2006, pages 61 - 62, XP008182445
- LAVERDIERE ISABELLE ET AL.: 'Liquid Chromatography-Coupled Tandem Mass Spectrometry Based Assay to Evaluate Inosine-5'- monophosphate Dehydrogenase Activity in Peripheral Blood Mononuclear Cells from Stem Cell Transplant Recipients' ANAL CHEM vol. 84, no. 1, 2012, pages 216 - 223, XP055295182
- ZHANG TONG ET AL.: 'Evaluation of Coupling Reversed Phase, Aqueous Normal Phase, and Hydrophilic Interaction Liquid Chromatography with Orbitrap Mass Spectrometry for Metabolomic Studies of Human Urine' ANAL CHEM vol. 84, no. 4, 2012, pages 1994 - 2001, XP055295184

**Description**

**BACKGROUND OF THE INVENTION**

1. Field of the Invention

The present invention relates to a method for predicting an effective dose of 5-hydroxy-1H-imidazole-4-carboxamide or a salt thereof or a hydrate thereof for a myelodysplastic syndrome patient. The present invention further relates to a method for predicting whether or not a myelodysplastic syndrome patient is sensitive to a treatment using 5-hydroxy-1H-imidazole-4-carboxamide or a salt thereof or a hydrate thereof. The present invention further relates to a method for determining amounts of xanthosine monophosphate in blood. The present invention further relates to an apparatus for predicting an effective dose of 5-hydroxy-1H-imidazole-4-carboxamide for a myelodysplastic syndrome patient, and an apparatus for predicting whether or not a myelodysplastic syndrome patient is sensitive to a treatment using 5-hydroxy-1H-imidazole-4-carboxamide or a salt thereof or a hydrate thereof. The present invention further relates to a treatment agent and a treating method for use in the treatment of a myelodysplastic syndrome patient who has been predicted to be sensitive to a treatment using 5-hydroxy-1H-imidazole-4-carboxamide or a salt thereof or a hydrate thereof.

2. Description of the Related Art

5-Hydroxy-1H-imidazole-4-carboxamide (hereinafter, referred to as "Compound A") or a salt thereof or a hydrate thereof is known to be useful in myelodysplastic syndrome (MDS) (Cancer and Chemotherapy, 1989, Vol. 16, No. 1, pp. 123-130). Compound A or a salt thereof or a hydrate thereof is converted *in vivo* into ribosyl monophosphate (RMP) by adenine phosphoribosyltransferase (APRT), and inhibits inosine-5'-monophosphate dehydrogenase (IMPDH). IMPDH is an enzyme that produces xanthosine monophosphate (XMP) from inosine monophosphate (IMP).

As an IMPDH inhibitor, an immunosuppressive agent, such as mycophenolic acid or mycophenolate mofetil, is known. Regarding these immunosuppressive agents, a method of evaluating the degree of inhibition by an IMPDH inhibitor has been reported which includes adding IMP *in vitro* to the cell lysate of peripheral blood mononuclear cells (PBMC), which is a fraction containing lymphocytes obtained from blood, to allow an enzymatic reaction by IMPDH, and determining an amount of XMP (Analytical Chemistry, 2012, Vol. 84, pp. 216-223).

For the analysis of nucleotides, methods using hydrophilic interaction liquid chromatography-mass spectrometry (HILIC-MS) and aqueous normal phase chromatography-mass spectrometry have been reported (Analytical Chemistry, 2012, Vol. 84, pp. 1994-2001). Moreover, for the analysis of XMP, methods involving reversed-phase chromatography using an ion pair reagent and ion exchange chromatography have been reported.

XMP produced by IMPDH is further converted by an *in vivo* enzymatic reaction into guanosine monophosphate (GMP), guanosine diphosphate (GDP), and guanosine triphosphate (GTP) which in turn serve as a material for the synthesis of nucleic acids (Stryer Biochemistry (7th edition), 2013, pp. 689-693).

Sombogaard, 2010, thesis Erasmus Universiteit Rotterdam, 1-224 discloses a mycophenolic acid or mycophenolate mofetil (MMF) and the therapeutic uses thereof. US 4,753,935 discloses a morpholino ethyl ester of mycophenolic acid and some ester derivatives of the phenolic hydroxyl group and the use thereof as immunosuppressive and anti-inflammatory agents.

**SUMMARY OF THE INVENTION**

MDS is a disease in which dysplasia of blood cell morphology and hematocytopenia may be recognized, and therapeutic effects of a therapeutic agent for this are evaluated mainly by hematologic remission and improvement. However, since it takes time for the effects of a therapeutic agent to be expressed, it is difficult to monitor the effects of a therapeutic agent over a short time. The method described Analytical Chemistry, 2012, Vol. 84, pp. 216-223 requires complicated operations and time in the fractionation of PBMC. In addition, this method is a method in which non-endogenous IMP is added for evaluation, and does not accurately reflect the degree of IMPDH inhibition *in vivo.*

A variety of attempts have been made to analyze nucleotides. However, because nucleotides are hydrophilic low-molecular weight compounds, there is a difficulty of retention thereof in reversed-phase chromatography commonly used for analysis. Moreover, since nucleotides have a phosphate group, they are susceptible to occurrence of adsorption and/or tailing at the time of separation analysis. For these reasons, quantification of endogenous nucleotides is difficult. In addition, there are many types having similar mass and structure in the metabolites of nucleotides. Since these metabolites exhibit very similar behavior and retention time in liquid chromatography (LC) in many cases, it is difficult to quantitatively distinguish them by liquid chromatography-mass spectrometry (LC-MS). Analytical Chemistry, 2012, Vol. 84, pp. 216-223 has reported the analysis of XMP. However, this method focuses on XMP-rich samples and is not an analysis of endogenous XMP which is present in trace amounts.

It is an object of the present invention to provide a method for predicting an effective dose of Compound A or a salt thereof or a hydrate thereof for an MDS patient, in a simple operation and a short time.

It is another object of the present invention to provide a method for predicting whether or not an MDS patient is sensitive to a treatment using Compound A or a salt thereof or a hydrate thereof, in a simple operation and a short time.

It is a further object of the present invention to provide a method for accurately determining amounts of XMP which is present in trace amounts in blood.

It is a still further object of the present invention to provide an apparatus for predicting an effective dose of Compound A or a salt thereof or a hydrate thereof for an MDS patient, in a simple operation and a short time, and an apparatus for predicting whether or not an MDS patient is sensitive to a treatment using Compound A or a salt thereof or a hydrate thereof.

It is yet another object of the present invention to provide a treatment agent and a method for treating a patient, for use in the treatment of an MDS patient who has been predicted to be sensitive to a treatment using Compound A or a salt thereof or a hydrate thereof.

As a result of intensive studies to solve the above problems, the present inventors have found that an effective dose of Compound A or a salt thereof or a hydrate thereof for an MDS patient, and the sensitivity of an MDS patient to a treatment using Compound A or a salt thereof or a hydrate thereof can be predicted by determining a variation in amounts of XMP in blood. Furthermore, the present inventors have found that by setting the determining conditions in MS, it is possible to accurately determine amounts of XMP which is present in trace amounts in blood. The present invention has been completed based on these findings.

That is, according to the present invention, the following inventions are provided.

[1] A method for predicting an effective dose of Compound A or a salt thereof or a hydrate thereof for an MDS patient, comprising:

(a) a step of determining (i) amounts of XMP in whole blood collected from the patient before the administration of Compound A or a salt thereof or a hydrate thereof, and (ii) amounts of XMP in the whole blood collected from the patient after the administration of Compound A or a salt thereof or a hydrate thereof, or amounts of XMP in the whole blood obtained by bringing the blood collected from the patient before the administration of Compound A or a salt thereof or a hydrate thereof into contact with Compound A or a salt thereof or a hydrate thereof;

(b) a step of determining a variation of XMP from the amounts of XMP obtained in step (a); and

(c) a step of predicting an effective dose of Compound A or a salt thereof or a hydrate thereof from the variation of XMP obtained in step (b),

in which the step of determining amounts of XMP includes

(a1) a step of determining XMP in whole blood in two different determining conditions by MS,

(a2) a step of determining an ionic strength ratio of XMP in whole blood in two different determining conditions obtained in step (a1), and

(a3) a step of checking whether the ionic strength ratio of XMP in whole blood obtained in step (a2) is within a predetermined range,

in which the two different determining conditions are detection ions and/or ionization conditions in MS, and wherein step (a) includes the following steps:

(a1) a step of determining xanthosine monophosphate in whole blood in two different determining conditions by mass spectrometry;

(a2) a step of determining an ionic strength ratio of xanthosine monophosphate in whole blood in two different determining conditions obtained in step (al); and

(a3) a step of checking whether the ionic strength ratio of xanthosine monophosphate in whole blood obtained in step (a2) is less than 1,

wherein the two different determining conditions are detection ions and/or ionization conditions in mass spectrometry, wherein the ionic strength ratio is calculated as follows:

ionic strength ratio = (strength of ions under the other determining condition)/(strength of product ion of 97.0 (m/z), when 365.0 (m/z) is taken as a precursor ion).

[2] The method according to [1], in which the step of determining amounts of XMP is a step carried out using conditions in which contaminants and XMP in blood can be quantitatively distinguished.

[3] The method according to any one of [1] or [2], in which the step of determining amounts of XMP is a step of determining amounts of XMP by mass spectrometry (MS).

[4] The method according to [1], in which step (a1) is a step of determining XMP in whole blood in two different determining conditions by LC-MS.

[5] The method according to [4], in which the pH value of the mobile phase used for liquid chromatography (LC) is 7 to 11.

[6] The method according to [4] or [5], in which the salt concentration of the mobile phase used for LC is 5 mmol/L to 300 mmol/L.

[7] The method according to any one of [4] to [6], in which the mobile phase used for LC is a mobile phase including an aqueous ammonium bicarbonate solution.

[8] The method according to any one of [4] to [7], in which the LC is hydrophilic interaction liquid chromatography (HILIC).

[9] The method according to any one of [1] to [8], in which step (c) is a step including

(c1) a step of expressing the relationship between the post-administration time or contact time of Compound A or a salt thereof or a hydrate thereof and the variation of XMP obtained in step (b) in terms of a function therebetween, and
(c2) a step of predicting an effective dose of Compound A or a salt thereof or a hydrate thereof from the function obtained in step (c1).

[10] The method according to [9], in which step (c2) is a step including a step of predicting that the dose of Compound A or a salt thereof or a hydrate thereof at which a variation of XMP is greater than or equal to a predetermined variation in a given treatment period is an effective dose.

[11] The method according to [9], in which step (c2) is a step including a step of predicting that the dose of Compound A or a salt thereof or a hydrate thereof at which a variation of XMP is greater than or equal to a predetermined variation in a period longer than or equal to a specified proportion of a given treatment period is an effective dose.

[12] A method for predicting whether or not an MDS patient is sensitive to a treatment using Compound A or a salt thereof or a hydrate thereof, comprising:

(a) a step of determining (i) amounts of XMP in the blood collected from the patient before the administration of Compound A or a salt thereof or a hydrate thereof, and (ii) amounts of XMP in the blood collected from the patient after the administration of Compound A or a salt thereof or a hydrate thereof, or amounts of XMP in the blood obtained by bringing the blood collected from the patient before the administration of Compound A or a salt thereof or a hydrate thereof into contact with Compound A or a salt thereof or a hydrate thereof; which includes the above-defined steps (a1) to (a3);
(b) a step of determining a variation of XMP from the amounts of XMP obtained in step (a); and
(c) a step of predicting whether or not a patient is sensitive to a treatment using Compound A or a salt thereof or a hydrate thereof from the variation of XMP obtained in step (b).

[13] The method according to [12], in which the step of determining amounts of XMP is a step carried out using conditions in which contaminants and XMP in blood can be quantitatively distinguished.

[14] The method according to any one of [13] or [13], in which the step of determining amounts of XMP is a step of determining amounts of XMP by MS.

[15] The method according to [12], in which step (a1) is a step of determining XMP in blood in two different determining conditions by LC-MS.

[16] The method according to [15], in which the pH value of the mobile phase used for LC is 7 to 11.

[17] The method according to [15] or [16], in which the salt concentration of the mobile phase used for LC is 5 mmol/L to 300 mmol/L.

[18] The method according to any one of [15] to [17], in which the mobile phase used for LC is a mobile phase including an aqueous ammonium bicarbonate solution.

[19] The method according to any one of [15] to [18], in which the LC is HILIC.
[20] The method according to any one of [15] to [19], in which the two different determining conditions are detection ions and/or ionization conditions in MS.
[21] The method according to any one of [12] to [20], in which step (c) is a step including

(c1) a step of expressing the relationship between the post-administration time or contact time of Compound A or a salt thereof or a hydrate thereof and the variation of XMP obtained in step (b) in terms of a function therebetween, and
(c2) a step of predicting whether or not a patient is sensitive to a treatment using Compound A or a salt thereof or a hydrate thereof from the function obtained in step (c1).

[22] The method according to [21], in which step (c2) is a step including a step of predicting that a patent is sensitive to a treatment using Compound A or a salt thereof or a hydrate thereof, in the case where the variation of XMP is greater than or equal to a predetermined variation in a given treatment period.
[23] The method according to [21], in which step (c2) is a step including a step of predicting that a patent is sensitive to a treatment using Compound A or a salt thereof or a hydrate thereof, in the case where the variation of XMP is greater than or equal to a predetermined variation in a period longer than or equal to a specified proportion of a given treatment period.

The determining method of the present invention can accurately determine amounts of XMP which is present in trace amounts in blood.

The prediction apparatus of the present invention is an apparatus using blood as a sample, and can thereby be used to predict an effective dose of Compound A or a salt thereof or a hydrate thereof for an MDS patient in a simple operation and a short time. Further, it can be used for predicting whether or not an MDS patient is sensitive to a treatment using Compound A or a salt thereof or a hydrate thereof in a simple operation and a short time.

Since the treatment agent and the treatment method of the present invention are used in the treatment of an MDS patient who has been predicted to be sensitive to a treatment using Compound A or a salt thereof or a hydrate thereof, it is possible to avoid unnecessary administration to a patient who exhibits no sensitivity to Compound A or a salt thereof or a hydrate thereof.

The method and the treatment agent of the present invention may be applied to kits for treating MDS patients, according to conventional methods.

## BRIEF DESCRIPTION OF THE DRAWINGS

Fig. 1 shows the antitumor effect of Compound A.
Fig. 2 shows the results of the PK/PD analysis (PK parameter: Cmax).
Fig. 3 shows the results of the PK/PD analysis (PK parameter: Time above IC50).
Fig. 4 shows the results of the PK/PD analysis (PK parameter: AUC0-48).
Fig. 5 shows the determination results of XMP in the XMP standard and human blood (sample) (determining conditions: A and B).
Fig. 6 shows the determination results of XMP in the XMP standard and human blood (sample) (determining conditions: C and D).
Fig. 7 shows the determination results of XMP in the XMP standard and human blood (sample) (determining conditions: A' and B').
Fig. 8 shows the determination results of XMP in the human blood (LC conditions: a and b).

## DESCRIPTION OF THE PREFERRED EMBODIMENTS

Hereinafter, the present invention will be described in detail. Further, the wording "(a lower limit) to (an upper limit)" means to include the former number indicating the lower limit of the range and the latter number indicating the upper limit thereof.

The term "treatment" means the prevention or treatment of a disease.

The term "treatment agent" means a substance that is used for the purpose of prevention or treatment of a disease.

The term "treatment period" means a period for applying a treatment.

The ionic strength includes, for example, the strength of the corresponding ion on the peak area or MS spectrum obtained from the chromatogram in LC-MS.

The term "XMP standard" means a preparation which is used when determining XMP in blood. The XMP standard may be, for example, a sodium salt of xanthosine monophosphate which is commercially available.

(1) Method for predicting effective dose of Compound A or salt thereof or hydrate thereof for MDS patient, and method for predicting whether or not MDS patient is sensitive to treatment using Compound A or salt thereof or hydrate thereof

In the method according to the present invention, step (a) includes determining (i) amounts of XMP in the blood collected from the patient before the administration of Compound A or a salt thereof or a hydrate thereof, and (ii) amounts of XMP in the blood collected from the patient after the administration of Compound A or a salt thereof or a hydrate thereof, or amounts of XMP in the blood obtained by bring the blood collected from the patient before the administration of Compound A or a salt thereof or a hydrate thereof into contact with Compound A or a salt thereof or a hydrate thereof.

Examples of the salt of Compound A include commonly known salts at a basic group or an acidic group. Examples of the salt of a basic group may include salts with mineral acids such as hydrochloric acid, hydrogen bromide, phosphoric acid, and sulfuric acid; salts with organic carboxylic acids such as tartaric acid, formic acid, acetic acid, fumaric acid, maleic acid, citric acid, trichloroacetic acid, and trifluoroacetic acid; and salts with sulfonic acids such as methanesulfonic acid, benzenesulfonic acid, p-toluenesulfonic acid, mesitylenesulfonic acid, and naphthalenesulfonic acid. Examples of the salt of an acidic group may include salts with alkali metals such as sodium and potassium; salts with alkaline earth metals such as calcium and magnesium; ammonium salts; and salts with nitrogen-containing organic bases such as trimethylamine, triethylamine, tributylamine, trometamol, pyridine, N,N-dimethylaniline, N-methylpiperidine, N-methylmorpholine, diethylamine, dicyclohexylamine, procaine, dibenzylamine, N-benzyl-$\beta$-phenethylamine, and N,N'-dibenzylethylenediamine. Among the aforementioned salts, preferred salts of Compound A are pharmacologically acceptable salts thereof.

Compound A or a salt thereof or a hydrate thereof can be prepared by the method described in, for example, JP1978-32124A (JP-S53-32124A), JP1983-24569A (JP-S58-24569A), WO2009/035168A or WO2013/047758A.

A feature of the present invention is the use of amounts of XMP in blood as an indicator. It is desirable to evaluate the IMPDH inhibitory effect using bone marrow cells of the bone marrow which is the lesion, in order to accurately predict an effective dose of a drug to MDS and the sensitivity to a drug. However, since the extraction of bone marrow cells suffers from heavy physical and mental burden given to a patient, the extraction frequency is limited. For this reason, the bone marrow cells necessary for the evaluation of IMPDH inhibitory effect are conventionally unavailable. In the present invention, it has been found for the first time that the IMPDH inhibitory effect of Compound A or a salt thereof or a hydrate thereof is equivalent in bone marrow cells and peripheral blood cells. This makes it possible to predict the IMPDH inhibitory effect on bone marrow cells from the IMPDH inhibitory effect on blood cells, and therefore it has become possible to predict an effective dose of a drug to MDS and the susceptibility to a drug. Blood used in the present invention is not particularly limited, and may be, for example, peripheral blood, blood which is pooled in bone marrow, spleen or liver, a lymphatic fluid, a tissue fluid or umbilical cord blood, preferably peripheral blood. Collection of blood from a patient can be carried out by conventional methods well known to those skilled in the art. Further, the blood used in the present invention is whole blood from the viewpoint of carrying out the prediction in a simple operation and a short time.

Step (a) in the present invention can be carried out in any one of the following two embodiments. The first embodiment is an embodiment of determining amounts of XMP in the blood collected from the patient before the administration of Compound A or a salt thereof or a hydrate thereof and amounts of XMP in the blood collected from the patient after the administration of Compound A or a salt thereof or a hydrate thereof.

The second embodiment is an embodiment of determining amounts of XMP in the blood collected from the patient before the administration of Compound A or a salt thereof or a hydrate thereof and amounts of XMP in the blood obtained by bringing the blood collected from the patient before the administration of Compound A or a salt thereof or a hydrate thereof into contact with Compound A or a salt thereof or a hydrate thereof.

In a first embodiment, the Compound A or a salt thereof or a hydrate thereof administered to a patient exerts its action in contact with blood cells in the body of the patient. In a second embodiment, by bringing the blood collected from the patient before the administration of Compound A or a salt thereof or a hydrate thereof into contact with Compound A or a salt thereof or a hydrate thereof *in vitro,* the Compound A or a salt thereof or a hydrate thereof exerts its action on blood cells.

In the present invention, amounts of XMP can be determined, for example, by MS. Prior to determination by MS, a blood sample may be subjected to crude separation of the sample, such as protein removal, or may be subjected to separation of components in the sample, such as by LC.

Because proteins, peptides and lipids contained in blood are typical interfering components of MS determination, these interfering components may be previously removed by the crude separation of the sample. For example, the crude separation may be carried out by fractionation processing (such as fractionation of cellular components by centrifugation, and removal of liquid components and/or red blood cells by hemolysis operation), filtration, processing of removed proteins, liquid-phase extraction and/or solid-phase extraction. Further, the crude separation of the sample, along with the separation and detection operations of components to be described later, may also be carried out on-line by pre-column or column switching, and the like.

Further, in the present invention, after LC separation of components in a sample, the sample is preferably supplied to an MS instrument.

As a method for separating components in the sample, it is possible to employ HILIC, reversed-phase chromatography (RP), chromatography using an ion pair reagent or ion exchange chromatography. Modes and determining conditions are not particularly limited as long as required separation and performance are obtained and elution can be carried out with a mobile phase composition that can be introduced into MS. A separation method such as capillary electrophoresis or gas chromatography may also be used.

The contaminants that may be detected in the determination of XMP may include, for example, a compound having a mass similar to that of XMP, a compound that produces the compound having a mass similar to that of XMP by degradation or the like and/or a compound having an elution time of LC similar to that of XMP. The compound having a mass similar to that of XMP may be, for example, an isotope of GMP. The compound that produces the compound having a mass similar to that of XMP by degradation or the like may be, for example, an isotope of GDP and an isotope of GTP. The compound having an elution time of LC similar to that of XMP may be, for example, reduced nicotinamide adenine dinucleotide phosphate (NADPH).

The step of determining amounts of XMP by MS is preferably carried out using conditions in which contaminants and XMP in blood can be quantitatively distinguished. It has been found through the review of the present inventors that the contaminants that may be detected in the determination of XMP (mass 364.04) include isotopes (mass 364.06 and mass 364.06) of GMP (mass 363.06) having a similar structure and a mass smaller by 1, isotopes of the in-source decomposition product of GDP and GTP (resulting in GMP by decomposition in the ionization moiety), and divalent ions derived from NADPH.

During the LC-MS determination, it is preferred to avoid the influence of contaminants and selectively detect XMP. In the present invention, by establishing a method of verifying whether XMP is detectable with sufficient specificity, it has become possible to selectively and conveniently quantify XMP with high sensitivity even under the conditions where a wide variety of and a large amount of contaminants are present.

The step of determining amounts of XMP is a step including

(a1) a step of determining XMP in blood in two different determining conditions by MS,
(a2) a step of determining an ionic strength ratio of XMP in blood in two different determining conditions obtained in step (a1), and
(a3) a step of checking whether the ionic strength ratio of XMP in blood obtained in step (a2) is within a predetermined range.

The details of the XMP determining method including (a1) to (a3) will be described later herein.

The quantification of XMP may be carried out by a known quantitative method, such as a calibration curve method (external standard method, standard addition method or internal standard method), or an isotope dilution method, and the quantitative method is not particularly limited.

In the present invention, subsequently to step (a), step (b) carries out a step of determining a variation of XMP from the amounts of XMP obtained in step (a).

The method of determining the variation of XMP is not particularly limited and the variation of XMP may be calculated, for example, according to the following equation.

$$\text{Variation (\%)} = (1 - (\text{amount of XMP after administration of Compound A or a salt thereof or a hydrate thereof/amount of XMP prior to administration of Compound A or a salt thereof or a hydrate thereof})) \times 100$$

or

$$\text{Variation (\%)} = (1 - (\text{amount of XMP after bringing into contact with Compound A or a salt thereof or a hydrate thereof/amount of XMP prior to bringing into contact with Compound A or a salt thereof or a hydrate thereof})) \times 100$$

In addition, as the amount of XMP, a value per unit blood volume may also be used, but a value per unit blood cell number is preferably used.

In the present invention, subsequently to step (b), step (c) predicts an effective dose of Compound A or a salt thereof or a hydrate thereof from the variation of XMP obtained in step (b), or predicts whether or not a patient is sensitive to a treatment using Compound A or a salt thereof or a hydrate thereof from the variation of XMP obtained in step (b).

For example, step (c) can be carried out by (c1) a step of expressing the relationship between the post-administration time of Compound A or a salt thereof or a hydrate thereof and the variation of XMP obtained in step (b) in terms of a function therebetween, and (c2) a step of predicting an effective dose of Compound A or a salt thereof or a hydrate thereof from the function obtained in step (c1). In addition, step (c) can be carried out by (c1) a step of expressing the relationship between the post-administration time of Compound A or a salt thereof or a hydrate thereof and the variation of XMP obtained in step (b) in terms of a function therebetween, and (c2) a step of predicting whether or not a patient is sensitive to a treatment using Compound A or a salt thereof or a hydrate thereof from the function obtained in step (c1).

Further, step (c) can be carried out by (c1) a step of expressing the relationship between the contact time of Compound A or a salt thereof or a hydrate thereof and the variation of XMP obtained in step (b) in terms of a function therebetween, and (c2) a step of predicting an effective dose of Compound A or a salt thereof or a hydrate thereof from the function obtained in step (c1). In addition, step (c) can be carried out by (c1) a step of expressing the relationship between the contact time of Compound A or a salt thereof or a hydrate thereof and the variation of XMP obtained in step (b) in terms of a function therebetween, and (c2) a step of predicting whether or not a patient is sensitive to a treatment using Compound A or a salt thereof or a hydrate thereof from the function obtained in step (c1).

Step (c2) predicts that the dose of Compound A or a salt thereof or a hydrate thereof at which the variation of XMP is greater than or equal to a predetermined variation (decline rate) in a given treatment period is an effective dose.

In addition, step (c2) predicts that a patient is sensitive to a treatment using Compound A or a salt thereof or a hydrate thereof in the case where the variation of XMP is greater than or equal to a predetermined variation (decline rate) in a given treatment period.

The predetermined treatment period may be appropriately selected depending on the species of organism. For example, the given treatment period may be from one day to two months, or may also be a period longer than or equal to a specified proportion of the given treatment period.

The period longer than or equal to a specified proportion of the given treatment period may be appropriately selected depending on the species of organism, and for example, it may be a period longer than or equal to 40% of the given treatment period.

The predetermined variation may be appropriately selected depending on the species of organism, and for example, it may be 45% or more (preferably 50% or more, and more preferably 55% or more).

For example, it can be predicted that in a period longer than or equal to 40% of the treatment period using Compound A or a salt thereof or a hydrate thereof, a dose such that the variation of XMP (decline rate) becomes 45% or more (preferably 50% or more, and more preferably 55% or more) is an effective dose. An effective dose of Compound A or a salt thereof or a hydrate thereof obtained here may be administered to an MDS patient.

Further, for example, it can be predicted that in a period longer than or equal to 40% of the treatment period using Compound A or a salt thereof or a hydrate thereof, a patient is sensitive to a treatment using Compound A or a salt thereof or a hydrate thereof in the case where the variation of XMP (decline rate) becomes 45% or more (preferably 50% or more, and more preferably 55% or more). The Compound A or a salt thereof or a hydrate thereof may be administered to a patient who has been predicted to be sensitive.

(2) Method for determining amounts of XMP in blood

The method of determining amounts of XMP in blood according to the present invention is a method including

    (a1) a step of determining XMP in blood in two different determining conditions by MS,
    (a2) a step of determining an ionic strength ratio of XMP in blood in two different determining conditions obtained in step (a1), and
    (a3) a step of checking whether the ionic strength ratio of XMP in blood obtained in step (a2) is within a predetermined range.

In step (a1), XMP is detected by MS. It has become possible to quantitatively distinguish between XMP and contaminants by carrying out the determination in two different determining conditions in step (a1), determining an ionic strength ratio of XMP in blood in two different determining conditions in step (a2), and checking whether the ionic strength ratio is within a predetermined range in step (a3).

In step (a1), it is preferred that the separation of a sample is carried out by LC and XMP is detected by MS (LC-MS).

In another embodiment, the method of determining amounts of XMP in blood according to the present invention may employ a method including

(a11) a step of determining an XMP standard and XMP in blood in two different determining conditions by MS,
(a12) a step of determining the ionic strength ratio of the XMP standard and the ionic strength ratio of XMP in blood in two different determining conditions obtained in (a11), and
(a13) a step of comparing the ionic strength ratio of the XMP standard and the ionic strength ratio of XMP in blood obtained in (a12).

Step (a12) determines the ionic strength ratio of the XMP standard and the ionic strength ratio of XMP in blood in two different determining conditions obtained in step (a11), and step (al3) compares the ionic strength ratio of the XMP standard and the ionic strength ratio of XMP in blood obtained in step (a12).

Here, in the case where the ionic strength ratio of the blood sample and the ionic strength ratio of the XMP standard are within a predetermined range, it can be determined that XMP in blood can be detected with high specificity. On the contrary, in the case where the ionic strength ratio of the blood sample and the ionic strength ratio of the XMP standard are outside a predetermined range, it is determined that the selectivity was insufficient in either or both of the conditions that took the ratio.

The LC may employ HILIC, RP, chromatography using an ion pair reagent or ion exchange chromatography, and preferably HILIC.

As the mobile phase used for LC, a mobile phase A of a basic aqueous solution and a mobile phase B containing an organic solvent can be preferably used.

The pH value of the mobile phase A is preferably 7 to 11, more preferably 8 to 11, even more preferably 8.4 to 10.4, and particularly preferably 9.0 to 9.8.

The pH value of the mobile phase A may be set according to the type of column to be used.

The salt concentration of the mobile phase used for LC is preferably 5 mmol/L to 300 mmol/L, and more preferably 20 mmol/L to 200 mmol/L.

The LC-MS determination typically employs a low salt concentration in order to give high sensitivity (e.g., 10 mmol/L), whereas the present invention employs a higher salt concentration as compared to typical salt concentration, whereby it became possible to separate XMP from contaminants.

The mobile phase A used for LC preferably contains an aqueous ammonium bicarbonate solution.

The organic solvent used in the mobile phase B may be, for example, acetonitrile, methanol, 2-propanol or ethanol, and is preferably acetonitrile or methanol and more preferably acetonitrile.

In one example of the present invention, XMP can be separated from contaminants by carrying out elution using a gradient method with an HILIC column (e.g., SeQuant ZIC-pHILIC, manufactured by Merck Ltd.) and a 50 mmol/L aqueous ammonium bicarbonate solution (adjusted to pH 9.4 by 25 mass% aqueous ammonia solution) and acetonitrile as mobile phases, with the time-dependent change in the ratio of mobile phase A to mobile phase B, or an isocratic method with a fixed ratio of mobile phase A to mobile phase B.

In the case where acetonitrile is used as mobile phase B, the concentration of acetonitrile in the mobile phase is preferably 20 volume% to 90 volume%, more preferably 35 volume% to 70 volume%, and still more preferably 65 volume%. The elution may be carried out by gradient elution with a variable ratio of mobile phase B to mobile phase A over time, but an isocratic method without changing the ratio of mobile phase A to mobile phase B is preferred in that the separation of contaminants is improved and analysis can be carried out in the condition where an injection volume is increased.

The present invention determines XMP in blood in two different determining conditions by MS.

In the MS method, the components of a sample are ionized and XMP-derived ions are selected and detected by an MS instrument. In order to detect selectively XMP from a large amount of and a wide variety of contaminants having mass and structures similar to those of XMP, it is preferred to secure high mass filter effects by means of (1) determination in an MS/MS mode, (2) use of high resolution MS instrument, or (3) use of both (1) and (2). Furthermore, the method of the present invention is characterized by setting the separation conditions and MS conditions (detection ions and/or ionization conditions), so that high selectivity can be achieved. In the method of the present invention, conditions in which XMP and contaminants can be quantitatively distinguished may be employed by the combination of the separation and MS detection. Whether the selectivity is sufficient can be evaluated by the method shown in step (a1), step (a2) and step (a3). In the present invention, if sufficient separation is achieved in LC, it is possible to accurately determine amounts of XMP in blood even in the case of using an MS instrument of low resolution commonly used in pharmacokinetics.

In the case of using a low resolution MS instrument, such as a triple quadrupole MS instrument, amounts of XMP can be determined by MS/MS determination such as Multiple Reaction Monitoring (MRM) mode to secure the specificity. It is preferred to determine in a plurality of conditions, such as detection of multiple MS/MS product ions resulting from the object to be determined. The condition providing a good specificity during analysis can be chosen, and the later-described validation of the specificity can be performed.

MS/MS determinations or MS determinations using a high resolution instrument such as Orbitrap MS (for example, Q-Exactive, manufactured by Thermo Co., Ltd.) and TOF MS (for example, 6550iFunnelQ-TOF, manufactured by Agilent Inc., and SYNAPT, manufactured by Waters Corporation) are capable of performing determinations with higher specificity. Although resolution is preferably high in the instrument, it is preferred to use an instrument from which the sensitivity and dynamic range required for the quantitative analysis of XMP can be obtained.

Step (a1) of the present invention determines XMP in blood in two different determining conditions by MS. In other words, the ions derived from XMP are determined in two different MS conditions. As for the two different determining conditions, it is preferred that detection ions and/or ionization conditions in MS are two different determining conditions. Specifically, the two different determining conditions may be, for example, determinations of multiple MS/MS product ions that occur in MS/MS determinations, determinations in different ionization conditions (such as the temperature of an ion source (gas temperature of a turbo heater) or the voltage of an ion introduction unit (orifice plate)), or determinations in a positive mode and a negative mode. For example, in the case of determining multiple MS/MS product ions, it is possible to use a combination of a product ion of 97.0 (m/z) when 365.0 (m/z) is taken as a precursor ion and a product ion of 153.0 (m/z) when 365.0 (m/z) is taken as a precursor ion, and a combination of a product ion of 97.0 (m/z) when 365.0 (m/z) is taken as a precursor ion and a product ion of 213.0 (m/z) when 365.0 (m/z) is taken as a precursor ion. In the case of determining amounts of XMP in whole blood, it is preferred to use a combination of a product ion of 97.0 (m/z) when 365.0 (m/z) is taken as a precursor ion and a product ion of 213.0 (m/z) when 365.0 (m/z) is taken as a precursor ion. In the case of determining amounts of XMP in hemolysis, it is preferred to use a combination of a product ion of 97.0 (m/z) when 365.0 (m/z) is taken as a precursor ion and a product ion of 153.0 (m/z) when 365.0 (m/z) is taken as a precursor ion.

In each condition, in order to assess whether or not it is possible to specifically detect XMP (for fear of detecting contaminants), the ionic strength ratio of XMP being within a predetermined range is checked by calculating ionic strength ratios in two determining conditions which are arbitrarily selected. That is, step (a2) of the present invention determines the ionic strength ratio of XMP in blood in two different determining conditions obtained in step (a1), and step (a3) checks whether the ionic strength ratio of XMP in blood obtained in step (a2) is within a predetermined range.

The ionic strength ratio is calculated, for example, by the following equation.

$$\text{Ionic strength ratio} = \frac{\text{(strength of ions under the other determining condition)}}{\text{(strength of product ion of 97.0 (m/z) when 365.0 (m/z) is taken as a precursor ion)}}$$

Here, as the strength of ions of the other condition, for example, the strength of the product ion of 153.0 (m/z) when 365.0 (m/z) is taken as a precursor ion or the strength of the product ion of 213.0 (m/z) when 365.0 (m/z) is taken as a precursor ion may be used.

Here, in the case where the ionic strength ratio of a blood sample is within a predetermined range, it can be determined that XMP in blood can be detected with high specificity. Conversely, in the case where the ionic strength ratio of a blood sample is outside a predetermined range, it is determined that the selectivity was insufficient in either or both of the conditions that took the ratio. The expression "within a predetermined range" is a case where the ionic strength ratio is less than 1 in the case of calculating the ionic strength ratio according to the above-described equation. When the intensity of ions of the other condition is the strength of the product ion of 213.0 (m/z) when 365.0 (m/z) is taken as a precursor ion, the ionic strength ratio is preferably 0.1 to 0.3. In order to improve the determination accuracy, verification may also be carried out in several combinations by changing the partner taking the ratio, with respect to certain conditions. For contaminants, such as GMP, that may be detected at the time of determination of XMP, it is possible to evaluate the presence or absence of overlap in this way. Verification of specificity is not limited to a peak area ratio in the chromatogram or an ionic strength ratio in the spectrum, and the verification method is not particularly limited as long as it is a verification method capable of fully evaluating the specificity. If the specificity is sufficient in a certain determining condition, it is possible to employ the results in such a determining condition. In the case where the specificity is insufficient in a certain determining condition, it may be sufficient to review the separation and detection conditions. For example, it has been found by the study of the present inventors that the detection of contaminants can be suppressed by changing the MS/MS product ions to be detected or lowering the temperature of an ion source (gas temperature of a turbo heater) and the voltage of the ion introduction unit (orifice plate).

Additionally, the expression "within a predetermined range" in the case of comparing the ionic strength ratio of XMP standard and the ionic strength ratio of XMP in blood is a case where the ionic strength ratio of XMP in blood is 0.5 to 1.5 of the ionic strength ratio of XMP standard.

As described above, it is possible to quantify XMP from the ions detected by the MS determination which was determined that the specificity is sufficient.

(3) Apparatus for predicting effective dose of Compound A or salt thereof or hydrate thereof, and apparatus for predicting sensitivity to Compound A or salt thereof or hydrate thereof

Further, an apparatus for predicting an effective dose of Compound A or a salt thereof or a hydrate thereof for an MDS patient, and an apparatus for predicting whether or not an MDS patient is sensitive to a treatment using Compound A or a salt thereof or a hydrate thereof are described. The apparatus is an apparatus including

(A) a means of determining (i) amounts of XMP in the blood collected from the patient before the administration of Compound A or a salt thereof or a hydrate thereof, (ii) amounts of XMP in the blood collected from the patient after the administration of Compound A or a salt thereof or a hydrate thereof, or amounts of XMP in the blood obtained by bringing Compound A or a salt thereof or a hydrate thereof into contact with the blood collected from the patient before the administration of Compound A or a salt thereof or a hydrate thereof,

(B) a means of determining a variation of XMP from the amounts of XMP obtained by the means described in (A), and

(C) a means of predicting an effective dose of Compound A or a salt thereof or a hydrate thereof or a means of predicting whether a patient is sensitive to a treatment using Compound A or a salt thereof or a hydrate thereof, from the variation of XMP obtained by the means described in (B).

The means described in (A) is not particularly limited as long as it is a means that can determine amounts of XMP in blood. For example, an MS instrument may be used. In addition, an apparatus with a combination of a separator and an MS instrument may also be used. For example, a high-performance liquid chromatography apparatus, a capillary electrophoresis apparatus or a gas chromatography apparatus may be used as the separator. Preferred is an LC-MS instrument.

The MS instrument typically includes a sample introducing unit, an ion source, an analysis unit, an ion detecting unit and a data processing unit.

The sample introducing unit is a portion for introducing a sample into an MS instrument. For example, with coupling of an MS instrument to a high-performance liquid chromatography apparatus, a capillary electrophoresis apparatus or a gas chromatography apparatus, it is also possible to introduce the sample into the MS instrument from these apparatuses.

The ion source is a portion for giving an electric charge to the sample, and the method for giving an electric charge is known such as an electron ionization (EI) method, a chemical ionization (CI) method, a field desorption (FD) method, a fast atom bombardment (FAB) method, a matrix-assisted laser desorption ionization (MALDI) method, an atmospheric pressure photoionization (APPI) method, an atmospheric pressure chemical ionization (APCI) method or an electrospray ionization (ESI) method. As the ion source used in LC-MS, APPI, APCI or ESI may be used. Preferred is ESI.

The analysis unit is a portion that separates the ionized sample. There have been known such as a magnetic deflection type, a quadrupole type, an ion trap type, a time-of-flight type, a Fourier transform ion cyclotron resonance type, and a tandem type.

The detecting unit is a portion which detects the ions sorted by the analysis unit after subjecting them to sensitizing with an electron multiplier or a microchannel plate.

The data processing unit is a portion for producing a mass spectrum from the obtained data.

As the means of determining a variation of XMP described in (B), it may be any means capable of determining the variation from the obtained amount of XMP and the above-described equation. For example, an electronic calculator (computer) with a calculation software or program may be used.

The means of predicting an effective dose of Compound A or a salt thereof or a hydrate thereof or the means of predicting whether or not a patient is sensitive to a treatment using Compound A or a salt thereof or a hydrate thereof, from the variation of XMP obtained by the means described in (B), each of which being described in (C), is not particularly limited as long as it is a means that can predict the effective dose or sensitivity from the variation of XMP based on the predetermined criteria. For example, it can be performed by using calculation software of the general purpose. For example, it is possible to use an electronic calculator (computer) having a program including a means of expressing the relationship between the post-administration time or contact time of Compound A or a salt thereof or a hydrate thereof and the variation of XMP in terms of a function therebetween, and a means of predicting an effective dose of Compound A or a salt thereof or a hydrate thereof or the presence or absence of sensitivity to a treatment using Compound A or a salt thereof or a hydrate thereof from the obtained function.

(4) Treatment agent for treating MDS in which active ingredient is Compound A, and method for treating MDS in which active ingredient is Compound A

Further, a treatment agent for treating MDS in which an active ingredient is Compound A, and a method for treating MDS in which an active ingredient is Compound A, each of which including a step of predicting whether or not an MDS patient is sensitive to a treatment using Compound A or a salt thereof or a hydrate thereof, are described.

The step of the present invention predicting whether or not an MDS patient is sensitive to a treatment using Compound A or a salt thereof or a hydrate thereof is as described above.

In the case where an MDS patient has been predicted to be sensitive to a treatment using Compound A or a salt thereof or a hydrate thereof, a treatment agent for treating MDS in which an active ingredient is Compound A is used in the treatment of the patient.

In the case where Compound A or a salt thereof or a hydrate thereof is used as a treatment agent for treating MDS, usually, pharmaceutical aids such as excipients, carriers and diluents used in the formulation may be mixed as appropriate. These aids may be administered orally or parenterally according to a conventional method, in the form of such as tablets, capsules, powders, syrups, granules, pills, suspensions, emulsions, solutions, powder preparations, suppositories, eye drops, nasal drops, ear drops, patches agent, ointments or injections. Moreover, although the route, dosage and frequency of administration may be appropriately selected depending on age, weight and condition of the patient, it is preferred to select a dosage such that in a duration of 40% or more of the treatment period, the variation of XMP (decline rate) is 45 % or more (preferably 50% or more, and more preferably 55% or more).

The present invention will be described in more detail with reference to the following examples, but the present invention is not limited thereto.

The ultrafiltration tube employed an UltrafreeMC-PLHCC 250/pk for Metabolome Analysis (manufactured by Human Metabolome Technologies Inc.).

The centrifugal evaporator employed a miVac Duo HV (manufactured by Genevac Inc.).

The PBS solution employed PBS, pH 7.4 (manufactured by Thermo Fisher Scientific Inc.).

EXAMPLES

Example 1 Cell growth inhibitory activity of Compound A

A 3/4 hydrate of Compound A was used as a test compound.

SKM-1 (available from National Institute of Biomedical Innovation JCRB Cell Bank) was used as a human myeloid leukemia cell line.

90 $\mu$L (5000 cells) of the human myeloid leukemia cell line SKM-1 was seeded onto a 96-well plate. 10 $\mu$L of a PBS solution of the test compound (0 $\mu$mol/L, 1 $\mu$mol/L, 3 $\mu$mol/L, 10 $\mu$mol/L, 30 $\mu$mol/L, 100 $\mu$mol/L, 300 $\mu$mol/L, 1000 $\mu$mol/L, 3000 $\mu$mol/L, or 10000 $\mu$mol/L in terms of Compound A) was added to the plate which was then incubated in 5% $CO_2$ at 37°C for 72 hours. 100 $\mu$L of CellTiter-Glo (manufactured by Promega) was added to each well to prepare a cell lysate.

The relative value of chemiluminescence intensity was calculated by a plate reader.

$IC_{50}$ value of Compound A was 21.5 $\mu$mol/L (2.73 $\mu$g/mL in terms of Compound A). Compound A exhibited an excellent antiproliferative activity.

Example 2 Drug Concentration Prediction by PK/PD Analysis (Pharmacokinetics/Pharmacodynamics Analysis)

(1) Antitumor Effects of Compound A

A 3/4 hydrate of Compound A was used as a test compound.

The SKM-1 cell was used as a human myeloid leukemia cell.

$5.0 \times 10^6$ human myeloid leukemia cells were subcutaneously transplanted into the right abdomen flank of female nude mice (BALB/cAJcl-nu/nu) to form a subcutaneous tumor. On day 9 post-transplantation, animals were assigned into 7 groups, each consisting of 10 mice. Table 1 shows the composition of groups. From day 10 post-transplantation, a control solvent (0.5 w/v% methyl cellulose 400 aqueous solution, hereinafter referred to as "0.5% MC".) or a test compound was intermittently administered orally (a total of five course in terms of 2 days dosing + four days washout as a course of drugs), and the tumor volume on day 39 post-transplantation was determined to evaluate the antitumor effects. The inhibition rate was calculated according to the following equation.

$$\text{Inhibition rate (\%)} = (1\text{-(tumor volume on day 39 post-transplantation)/(tumor volume of Group 1 on day 39 post-transplantation)}) \times 100$$

[Table 1]

| Group No. | Number of animals (Head) | Dose (mg/kg/day) | Dose (mg/kg/times) | Dosing frequency (times/day) |
|---|---|---|---|---|
| 1 | 10 | 0*1 | 0 | 3 |
| 2 | 10 | 240 | 240 | 1 |
| 3 | 10 | 240 | 120 | 2 |
| 4 | 10 | 240 | 80 | 3 |
| 5 | 10 | 240 | 40 | 6 |
| 6 | 10 | 120 | 20 | 6 |
| 7 | 10 | 480 | 160 | 3 |

*1) 0.5% MC administered

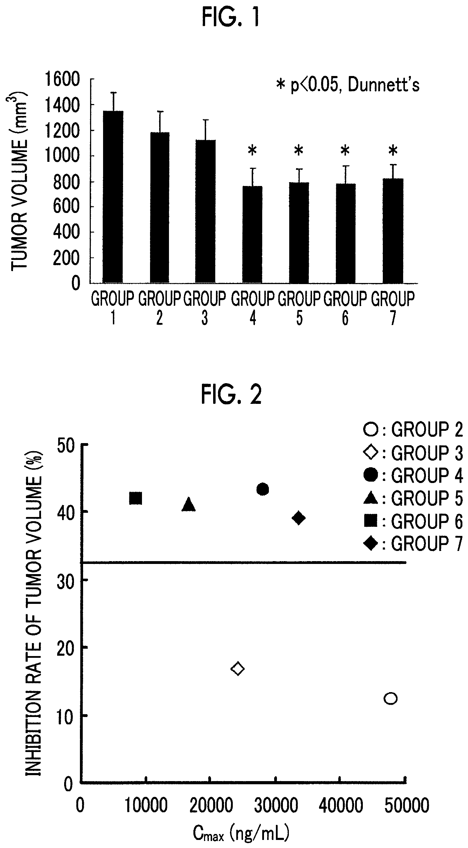

Figure 1 shows the tumor volume on day 39 post-transplantation.

Group 4, Group 5, Group 6 and Group 7 exhibited excellent antitumor effects.

(2) PK Test of Compound A

Non-fasting female mice (BALB/cAJcl) were given a single oral administration of the test compound (10 mg/kg, 40 mg/kg, 80 mg/kg and 160 mg/kg in terms of Compound A). After 5 minutes, 15 minutes, 30 minutes, 1 hour, 2 hours, 5 hours, 10 hours or 24 hours, heparinized blood was collected and then the concentration of Compound A in plasma was determined using LC/MS/MS to thereby calculate PK parameters ($t_{max}$, $C_{max}$, $t_{1/2}$ and $AUC_{0-24}$).

(3) PK/PD Analysis

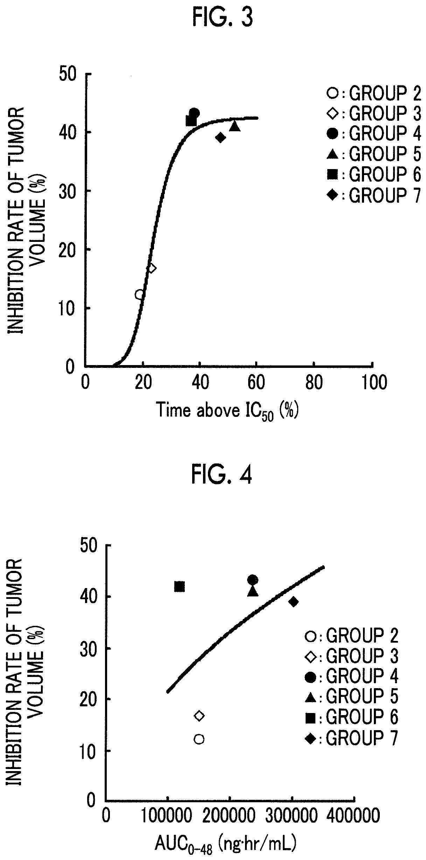

$V_1/F$, $K_{01}$, $K_{10}$, $K_{12}$ and $K_{21}$, pharmacokinetic parameters of the two-compartment model, were calculated from the plasma concentration course of Compound A when Compound A was given as a single oral administration. These parameters were used to predict the plasma concentration course of Compound A upon repeated administration in a variety of dosage regimen. In addition, since the linearity was recognized for from 10 mg/kg up to 80 mg/kg, the parameter of 10 mg/kg dosing was used to carry out the prediction at the time of repeated administration of 20 mg/kg, 40 mg/kg and 80 mg/kg. The parameter of 160 mg/kg was used to carry out the prediction for 120 mg/kg and 240 mg/kg. From the resulting plasma concentration course of Compound A, $C_{max}$ (ng/mL), $AUC_{0-48}$ (ng·time/mL) and Time above $IC_{50}$ (%, $IC_{50}$ value: 21.5 μmol/L (2.73 μg/mL in terms of Compound A)) were calculated. PK parameters ($C_{max}$, Time above $IC_{50}$ and $AUC_{0-48}$) were plotted on the X axis, and the inhibition rate of tumor volume was plotted on the Y axis. The results are shown in Figs. 2, 3 and 4.

Based on the fact that the correlation coefficient between $C_{max}$ and the inhibition rate is -0.5447, the correlation coefficient between Time above $IC_{50}$ and the inhibition rate is 0.9814, and the correlation coefficient between $AUC_{0-48}$ and the inhibition rate is 0.4684, the Time above $IC_{50}$ was a parameter that most correlates with the inhibition rate.

Also, the tumor volume was statistically significantly inhibited in groups in which the Time above $IC_{50}$ is 40% or more. Therefore, it was predicted to be important that the drug concentration in human plasma is maintained at a level of 2.73 μg/mL or higher in terms of Compound A, in the period of 40% or more of the treatment period.

Example 3 Quantitative Determination of XMP

(1-1) 450 μL of blood taken from healthy individuals by heparinized blood collection, and 50 μL of a PBS solution were added to the tube, followed by stirring in an incubator at 37°C for 8 hours.

(1-2) 400 μL of methanol was added to 100 μL of the sample obtained in (1-1). After stirring with a vortex mixer, 400 μL of chloroform was added and stirred with a vortex mixer, followed by addition of 120 μL of ultrapure water. After stirring with a vortex mixer, the reaction solution was centrifuged at 4°C and 10000 × g for 15 min. 400 μL of

the aqueous phase was recovered and added to an ultrafiltration tube, followed by centrifugation at 12°C and 9200 × g for 2 hours. The filtrates were combined and then dried under reduced pressure at 40°C for 2 hours by using a centrifugal evaporator. After drying, the resulting product was stored in a freezer at -80°C.

(1-3) The sample was dissolved in water, followed by centrifugation, and the supernatant was recovered to serve as a determination sample. The determination sample and XMP standard were subjected to LC-MS/MS. The XMP standard used was XMP (Xanthosine-5'-monophosphate, Sodium salt) (manufactured by Jena Bioscience). The configuration of an apparatus and determining conditions are shown below.

(LC-MS/MS)

LC: UFLC (manufactured by Shimadzu Corporation)
MS: QTRAP 5500 (manufactured by AB SCIEX)

(LC)

Column: SeQuant ZIC-pHILIC 5 μm, polymeric PEEK 150×2.1 mm metal-free HPLC column (manufactured by Merck Ltd.)

Column oven temperature: 40°C

Mobile phase A: 50 mmol/L of ammonium bicarbonate (adjusted to pH 9.4 by 25 mass% aqueous ammonia solution)

Mobile phase B: acetonitrile

Feeding conditions: 0.3 mL/min

The gradient cycles are shown in Table 2. % in Table 2 indicates volume%.

[Table 2]

| Time (min) | 0 | 5.83 | 5.84 | 9.83 | 9.84 | 13.83 |
|---|---|---|---|---|---|---|
| Mobile phase A (%) | 30 | 65 | 100 | 100 | 30 | 30 |
| Mobile phase B (%) | 70 | 35 | 0 | 0 | 70 | 70 |

(MS/MS)

Ionization: ESI positive (Electrospray ionization positive)

Scan mode: MRM (Multiple Reaction Monitoring)

The determining conditions are shown in Table 3.

[Table 3]

| Determining object | Determining conditions | Precursor ion (m/z) /Product ion (m/z) | Temperature (gas temperature of turbo heater) (°C) | Voltage of ion introduction unit (orifice plate) (V) |
|---|---|---|---|---|
| XMP | A B | 365.0/97.0 365.0/153.0 | 600 | 50 |

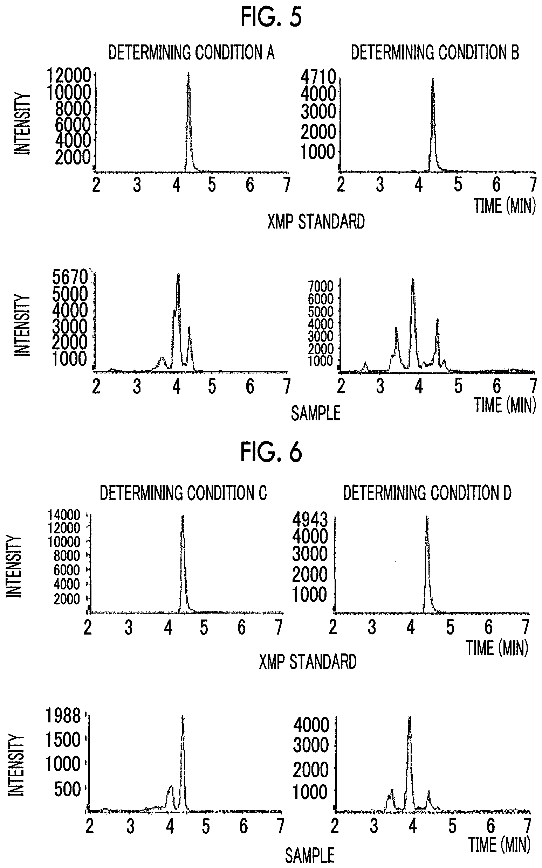

Fig. 5 shows the determination results obtained in (1-3).

(1-4) Analysis 1

Multiple adjacent peaks were observed in the chromatogram of the sample. The peak of XMP in the sample was estimated from the peak of XMP standard to calculate an area thereof. In order to verify the specificity, the ratio of the peak area in the determining condition A to the peak area in the determining condition B (B/A) was calculated. The results are shown in Table 4.

[Table 4]

| Determining conditions | Precursor ion (m/z)/Product ion (m/z) | Peak area | |
| --- | --- | --- | --- |
| | | XMP standard | Sample (human blood) |
| A | 365.0/97.0 | 83000 | 15000 |
| B | 365.0/153.0 | 29200 | 18700 |
| Peak area ratio (B/A) | | 0.4 | 1.2 |

The peak area ratio of the sample (B/A) was 1.2. Since the peak area ratio of the sample is 1 or more, it was determined that the specificity is insufficient in either or both of the determining conditions A and B (Contaminants are detected with overlapping).

(1-5) Analysis 2

In determining conditions C and D, XMP standard and the sample obtained in (3) were determined. The determining conditions C and D are shown in Table 5. The determination results of XMP standard and sample are shown in Fig. 6 and Table 6.

[Table 5]

| Determining object | Determining conditions | Precursor ion (m/z) /Product ion (m/z) | Temperature (gas temperature of turbo heater) (°C) | Voltage of ion introduction unit (orifice plate) (V) |
| --- | --- | --- | --- | --- |
| XMP | C | 365.0/97.0 | 350 | 20 |
| | D | 365.0/153.0 | | |

[Table 6]

| Determining conditions | Precursor ion (m/z)/Product ion (m/z) | Peak area | |
| --- | --- | --- | --- |
| | | XMP standard | Sample (human blood) |
| C | 365.0/97.0 | 94500 | 10300 |
| D | 365.0/153.0 | 33000 | 4540 |
| Peak area ratio (D/C) | | 0.3 | 0.4 |

Peak area ratio of the sample (D/C) was 0.4. From the point that the peak area ratio of the sample is less than 1, it was determined to be good specificity.

From this result, it was determined that amounts of XMP can be determined by means of determining conditions C and D.

(2-1) A sample was prepared in the same manner as those described in (1-1) to (1-3), except that the determination is carried out in the determining conditions shown in Table 7. Then, XMP of the sample was determined.

[Table 7]

| Determining object | Determining conditions | Precursor ion (m/z) /Product ion (m/z) | Temperature (gas temperature of turbo heater) (°C) | Voltage of ion introduction unit (orifice plate) (V) |
|---|---|---|---|---|
| XMP | A' B' | 365.0/97.0 365.0/213.0 | 350 | 20 |

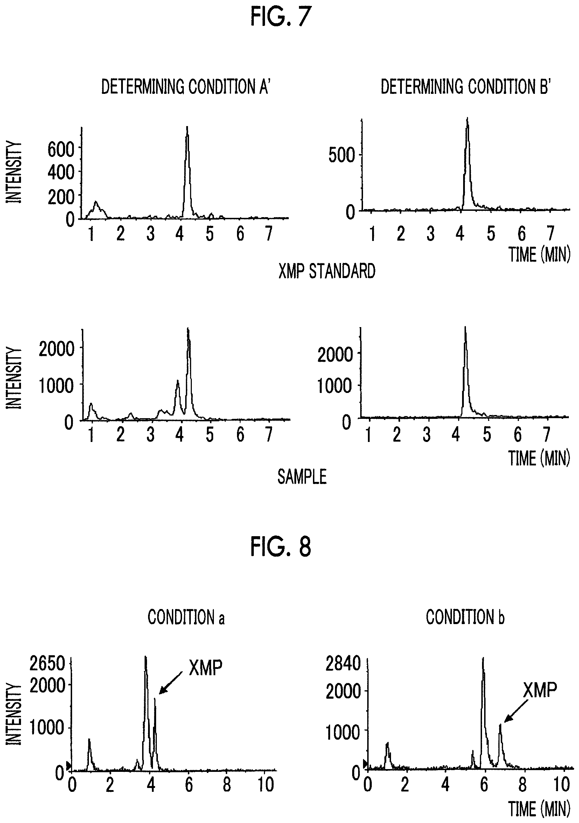

Fig. 7 shows the determination results obtained in (2-1)

(2-2) Analysis

In order to verify the specificity, the ratio of the peak area in the determining condition A' to the peak area in the determining condition B' (B'/A') was calculated. The results are shown in Table 8.

[Table 8]

| Determining conditions | Precursor ion (m/z)/Product ion (m/z) | Peak area | |
|---|---|---|---|
| | | XMP standard | Sample (human blood) |
| A' | 365.0/97.0 | 20000 | 25000 |
| B' | 365.0/213.0 | 6000 | 5000 |
| Peak area ratio (B'/A') | | 0.3 | 0.2 |

The peak area ratio of the sample (B'/A') was 0.2. Since the peak area ratio of the sample is within the range of 0.1 to 0.3, it was determined to be very good specificity.
Based on this result, it was determined that amounts of XMP can be determined by means of determining conditions A' and B'.

Example 4 XMP in human myeloid leukemia cells and XMP in blood cells of healthy human

A 3/4 hydrate of Compound A was used as a test compound.
K562 (available from Riken BioResource Center) was used as a human myeloid leukemia cell.

(1) $2 \times 10^7$ human myeloid leukemia cells were suspended in a culture flask containing 18 mL of an RPMI1640 medium (manufactured by Life Technologies, Inc.). 2 mL of a PBS solution of the test compound (0 μg/mL, 10 μg/mL, 100 μg/mL, or 1000 μg/mL in terms of Compound A) was added to the flask which was then incubated at 37°C under 5% $CO_2$ for 24 hours. After centrifugation at 20°C and 300 × g for 3 min, the supernatant was removed and suspended in 10 mL of a PBS solution. Then, after centrifugation at 4°C and 300 × g for 5 min, the supernatant was removed. After stirring with addition of 1000 μL of methanol, 500 μL of ultrapure water was added thereto, followed by further stirring. 600 μL of the reaction solution was divided into each of two tubes, and 400 μL of chloroform was added to each tube, The reaction solution was stirred and then centrifuged at 4°C and 10000 × g for 15 min. 400 μL of the aqueous phase was recovered and added to an ultrafiltration tube, followed by centrifugation at 12°C and 9200 × g for 2 hours. The filtrates were combined and then dried under reduced pressure at 40°C for 2 hours by using a centrifugal evaporator. After drying, the resulting product was stored in a freezer at -80°C.
(2) 450 μL of the blood collected from two healthy individuals by heparinized blood collection (Analyte A and Analyte B) was added to each of 1.5 mL tubes, and 50 μL of a PBS solution of the test compound (0 μg/mL, 10 μg/mL, 30 μg/mL, 100 μg/mL, 300 μg/mL, or 1000 μg/mL in terms of Compound A) for each concentration was added to each tube. The reaction mixture was stirred in an incubator at 37°C for 8 hours. Then, BD Pharm Lyse (555899, manufactured by Becton, Dickinson and Company, Inc.) was added to the blood (2 mL of BD Pharm Lyse was added relative to 0.2 mL of blood). After stirring with a vortex mixer, the reaction solution was protected from light and allowed to stand at room temperature for 15 min. After centrifugation at 20°C and 1000 × g for 5 min, the supernatant was removed and suspended in 10 mL of a PBS solution. After centrifugation at 4°C and 1000 × g for 5 min, the supernatant was removed, and 1000 μL of methanol was added to each tube, followed by stirring. Thereafter, 500 μL of ultrapure water was added thereto, followed by further stirring. 600 μL of the reaction solution was divided into each of two tubes, and 400 μL of chloroform was added to each tube. The reaction solution was stirred and then centrifuged at 4°C and 10000 × g for 15 min. 400 μL of the aqueous phase was recovered and added to an ultrafiltration tube, followed by centrifugation at 12°C and 9200 × g for 2 hours. The filtrates were combined and then dried under reduced pressure at 40°C for 2 hours by using a centrifugal evaporator. After drying, the resulting product was stored in a freezer at -80°C.

(3) The samples stored in (1) and (2) were returned to room temperature and 40 μL of ultrapure water was added to each sample. The mixture was stirred and centrifuged at 4°C and 21500 × g for 5 min. 35 μL of the supernatant was transferred to a vial. After storage at 4°C, XMP in samples was determined in the LC-MS/MS determination instrument, LC conditions and MS/MS conditions described in Example 3 (3).

(4)Amounts of XMP (%) were calculated assuming that the XMP value of a non-Compound A treated group (peak area value) is defined as 100%.

The results are shown in Table 9.

[Table 9]

| Concentration of Compound A (μg/mL) | Amount of XMP (%) | | |
|---|---|---|---|
| | Analyte A | Analyte B | K562 |
| 0 | 100 | 100 | 100 |
| 1 | 60 | 51 | 52 |
| 3 | 45 | 37 | - |
| 10 | 37 | 25 | 15 |
| 30 | 27 | 18 | - |
| 100 | 16 | 10 | 6 |

Variations of amounts of XMP in healthy person's blood cells and human myeloid leukemia cells were comparable to each other. It has been suggested that the variation of amounts of XMP with respect to Compound A in blasts of the bone marrow of MDS patients can be predicted by determining a variation thereof in blood cells of peripheral blood.

Example 5 XMP in blood cells after hemolysis and XMP in whole blood

A 3/4 hydrate of Compound A was used s a test compound.

(1) 450 μL of the blood collected from two healthy individuals by heparinized blood collection (Analyte A and Analyte B) was added to each tube, and 50 μL of a PBS solution of the test compound (0 μg/mL, 10 μg/mL, 30 μg/mL, 100 μg/mL, 300 μg/mL, or 1000 μg/mL in terms of Compound A) for each concentration was added to each tube. The reaction mixture was stirred in an incubator at 37°C for 8 hours.

(2-1) BD Pharm Lyse (555899, manufactured by Becton, Dickinson and Company, Inc.) was added to the blood sample obtained in (1) (2 mL of BD Pharm Lyse was added relative to 0.2 mL of blood). After stirring with a vortex mixer, the reaction solution was protected from light and allowed to stand at room temperature for 15 min. After centrifugation at 20°C and 1000 × g for 5 min, the supernatant was removed and suspended in 10 mL of PBS. After centrifugation at 20°C and 1000 × g for 5 min, the supernatant was removed and suspended in 10 mL of PBS. After centrifugation at 4°C and 1000 × g for 5 min, the supernatant was removed, and 1000 μL of methanol was added to each tube, followed by stirring. Thereafter, 500 μL of ultrapure water was added thereto, followed by further stirring. 600 μL of the reaction solution was divided into each of two tubes, and 400 μL of chloroform was added to each tube. The reaction solution was stirred and then centrifuged at 4°C and 10000 × g for 15 min. 400 μL of the aqueous phase was recovered and added to an ultrafiltration tube (UltrafreeMC-PLHCC 250/pk for Metabolome Analysis (manufactured by Human Metabolome Technologies)), followed by centrifugation at 12°C and 9200 × g for 2 hours. The filtrates were combined and then dried under reduced pressure at 40°C for 2 hours by using a centrifugal evaporator. After drying, the resulting product was stored in a freezer at -80°C.

(2-2) 400 μL of methanol was added to 100 μL of the blood sample obtained in (1). After stirring, 400 μL of chloroform was added, followed by further stirring. 120 μL of ultrapure water was added thereto, followed by stirring and then centrifugation at 4°C and 10000 × g for 15 min. 400 μL of the aqueous phase was recovered and added to an ultrafiltration tube, followed by centrifugation at 12°C and 9200 × g for 2 hours. The filtrate was dried under reduced pressure at 40°C for 2 hours by using a centrifugal evaporator. After drying, the resulting product was stored in a freezer at -80°C.

(3) The samples obtained in (2-1) and (2-2) were returned to room temperature and 40 μL of ultrapure water was added to each sample. The mixture was stirred and centrifuged at 4°C and 21500 × g for 5 min. 35 μL of the supernatant was transferred to a vial. After storage at 4°C, XMP in samples was determined in the LC-MS/MS determination instrument, LC conditions and MS/MS conditions described in Example 3 (3).

(4) Amounts of XMP (%) were calculated assuming that amounts of XMP in a non-Compound A treated group are defined as 100%.

The results are shown in Table 10.

[Table 10]

| Concentration of Compound A (μg/mL) | Amount of XMP (%) | | | |
|---|---|---|---|---|
| | Analyte A | | Analyte B | |
| | Hemolysis | Whole blood | Hemolysis | Whole blood |
| 0 | 100 | 100 | 100 | 100 |
| 1 | 60 | 57 | 51 | 44 |
| 3 | 45 | 53 | 37 | 27 |
| 10 | 37 | 47 | 25 | 15 |
| 30 | 27 | 26 | 18 | 11 |
| 100 | 16 | 15 | 10 | 9 |

The amount of XMP in blood cells after hemolysis and the amount of XMP in whole blood were almost comparable to each other. The amount of XMP in whole blood reflected the amount of XMP in blood cells.

Example 6 XMP in blood cells and XMP in bone marrow cells

A 3/4 hydrate of Compound A was used as a test compound.

(1) 0.5% MC or 0.5% MC of the test compound (100 mg/kg in terms of Compound A) was orally administered to female mice (BALB/cAJcl).

(2-1) Whole blood was collected from the postcava of animals under anesthesia by isoflurane inhalation at 1, 4, 8 or 24 hours post-dose, using a polypropylene syringe treated with an ethylenediaminetetraacetic acid disodium salt and a 25 gauge injection needle. BD Pharm Lyse (555899, manufactured by Becton, Dickinson and Company, Inc.) was added to the blood (2 mL of BD Pharm Lyse was added relative to 0.2 mL of blood). After stirring with a vortex mixer, the reaction solution was protected from light and allowed to stand at room temperature for 15 min. After centrifugation at 20°C and 1000 × g for 5 min, the supernatant was removed and suspended in 10 mL of a PBS solution. After centrifugation at 4°C and 1000 × g for 5 min, the supernatant was removed, and 1000 μL of methanol was added to each tube, followed by stirring. Thereafter, 500 μL of ultrapure water was added thereto, followed by further stirring. 600 μL of the reaction solution was divided into each of two tubes, and 400 μL of chloroform was added to each tube. The reaction solution was stirred and then centrifuged at 4°C and 10000 × g for 15 min. 400 μL of the aqueous phase was recovered and added to an ultrafiltration tube, followed by centrifugation at 12°C and 9200 × g for 2 hours. The filtrates were combined and then dried under reduced pressure at 40°C for 2 hours by using a centrifugal evaporator. After drying, the resulting product was stored in a freezer at -80°C.

(2-2) Right and left femurs of animals were collected under anesthesia by isoflurane inhalation at 1, 4, 8 or 24 hours post-dose, followed by cutting bone ends and centrifuging at 4°C and 3000 rpm for 1 min to recover bone marrow cells. The recovered bone marrow cells were suspended in 1 mL of PBS, and the number of cells was counted. After centrifugation at 4°C and 1000 × g for 5 min, the supernatant was removed, and 1000 μL of methanol was added to each tube, followed by stirring. Thereafter, 500 μL of ultrapure water was added thereto, followed by further stirring. 600 μL of the reaction solution was divided into each of two tubes, and 400 μL of chloroform was added to each tube. The reaction solution was stirred and then centrifuged at 4°C and 10000 × g for 15 min. 400 μL of the aqueous phase was recovered and added to an ultrafiltration tube, followed by centrifugation at 12°C and 9200 × g for 2 hours. The filtrates were combined and then dried under reduced pressure at 40°C for 2 hours by using a centrifugal evaporator. After drying, the resulting product was stored in a freezer at -80°C.

(3) The samples obtained in (2-1) and (2-2) were returned to room temperature, and 40 μL of ultrapure water was added to the hemolysis sample and 150 μL of ultrapure water was added to the bone marrow sample. The mixture was stirred and then centrifuged at 4°C and 21500 × g for 5 min. For the hemolysis sample, 35 μL of the supernatant was transferred to a vial. For the bone marrow sample, 140 μL of the supernatant was transferred to a vial. After storage at 4°C, XMP in samples was determined in the LC-MS/MS determination instrument, LC conditions and MS/MS conditions described in Example 3 (3).

(4) Amounts of XMP (%) were calculated assuming that amounts of XMP in a non-Compound A treated group are defined as 100%.

The results are shown in Table 11.

[Table 11]

| Time post-dose (hr) | Amount of XMP % | |
| --- | --- | --- |
| | Blood cell | Bone marrow cell |
| Non-treated | 100 | 100 |
| 1 | 3.4 | 8.7 |
| 4 | 7.2 | 16.5 |
| 8 | 26.4 | 25.9 |
| 24 | 70.7 | 82.7 |

Variations of XMP in blood cells and bone marrow cells were equivalent to each other. The variation of XMP with respect to Compound A in blood cells was suggested to reflect the variation in bone marrow cells.

From the results of Example 4, Example 5 and Example 6, the variation of XMP in peripheral whole blood was shown to reflect the variation of XMP in blasts of the bone marrow of MDS patients.

Example 7 XMP in human myeloid leukemia cells

A 3/4 hydrate of Compound A was used as a test compound.

SKM-1 was used as a human myeloid leukemia cell.

(1) $2 \times 10^7$ human myeloid leukemia cells were suspended in a culture flask containing 18 mL of an RPMI1640 medium (manufactured by Life Technologies, Inc.). 2 mL of a PBS solution of the test compound (0 μg/mL, 10 μg/mL, 30 μg/mL, 100 μg/mL, 300 μg/mL, or 1000 μg/mL in terms of Compound A) was added to the flask which was then incubated at 37°C under 5% $CO_2$ for 24 hours. After centrifugation at 20°C and 300 × g for 3 min, the supernatant was removed and suspended in 10 mL of a PBS solution. 10 μL of the suspension was aliquoted and cells were counted using TC10 (manufactured by BioRad). After centrifugation and 300 × g for 5 min, the supernatant was removed. After stirring with addition of 1000 μL of methanol, 500 μL of ultrapure water was added thereto, followed by further stirring. 600 μL of the reaction solution was divided into each of two tubes, and 400 μL of chloroform was added to each tube. The reaction solution was stirred and then centrifuged at 4°C and 10000 × g for 15 min. 400 μL of the aqueous phase was recovered and added to an ultrafiltration tube, followed by centrifugation at 12°C and 9200 × g for about 2 hours. The filtrates were pooled in one tube and then dried under reduced pressure at 40°C for 2 hours by using a centrifugal evaporator. After drying, the resulting product was stored in a freezer at -80°C.

(2) The samples obtained in (1) were returned to room temperature, to each of which 50 μL of 5 mmol/L ammonium formate containing 4 ng/μL of 2'-fluoro-2'-deoxycytidine monophosphate was then added, followed by stirring. 12.5 μL of the supernatant was transferred to a vial, and XMP in the samples was determined in the following determining conditions.

(LC-MS/MS)

LC: UFLC (manufactured by Shimadzu Corporation)

MS: QTRAP3200 (manufactured by AB-Sciex Inc.)

(LC)

Column: SeQuant ZIC-pHILIC 5 μm, polymeric PEEK 150×2.1 mm metal-free HPLC column (manufactured by Merck Ltd.)

Column oven temperature: 40°C

Mobile phase A: 10 mmol/L of ammonium bicarbonate (adjusted to pH 9.4 by 25 mass% aqueous ammonia solution)

Mobile phase B: acetonitrile

Feeding conditions: 0.5 mL/min

The gradient cycles are shown in Table 12. % in Table 12 indicates volume%.

[Table 12]

| Time (min) | 0 | 5.83 | 5.84 | 9.83 | 9.84 | 12.00 |
|---|---|---|---|---|---|---|
| Mobile phase A (%) | 30 | 65 | 100 | 100 | 30 | 30 |
| Mobile phase B (%) | 70 | 35 | 0 | 0 | 70 | 70 |

(MS/MS)

Ionization: ESI positive

Scan Mode: MRM

The determining conditions are shown in Table 13.

[Table 13]

| Precursor ion(m/z)/ Product ion(m/z) | Temperature (gas temperature of turbo heater) (°C) | Voltage of ion introduction unit (orifice plate) (V) |
|---|---|---|
| 365.0/213.0 | 500 | 50 |

(3) Amounts of XMP (%) were calculated according to the following equation, assuming that the XMP value of a non-Compound A treated group is defined as 100%.

$$\text{Amount of XMP (\%)} = ((\text{peak area of XMP})/((\text{peak area of 2'-fluoro-2'-deoxycytidine monophosphate}) \times (\text{number of cells})))/((\text{peak area of XMP of untreated group})/((\text{peak area of 2'-fluoro-2'-deoxycytidine monophosphate of untreated group}) (\text{number of cells of untreated group}))) \times 100$$

The decline rate of XMP (%) was calculated according to the following equation.

$$\text{Decline rate of XMP (\%)} = 100 - \text{amount of XMP (\%)}$$

The results are shown in Table 14.

[Table 14]

| Concentration of Compound A (μg/mL) | Amount of XMP (%) | Decline rate of XMP |
|---|---|---|
| 0 | 100 | 0 |
| 1 | 57 | 43 |
| 10 | 45 | 55 |
| 100 | 17 | 83 |

An $IC_{50}$ value in SKM-1 cells of Compound A is 2.73 μg/mL (Example 1). Therefore, the XMP decline rate in the vicinity of the $IC_{50}$ value was 43% to 55%.

From the results of Examples 2 and 7, it can be predicted that the dosage is effective when the XMP decline rate is 45% or more in the period of 40% or more of a treatment period.

Example 8 XMP, GMP and GTP in whole blood

A 3/4 hydrate of Compound A was used as a test compound.

(1) 450 μL of the blood collected from healthy individuals by heparinized blood collection was added to each tube, and 50 μL of a PBS solution of the test compound (0 μg/mL, 1 μg/mL, 10 μg/mL, or 100 μg/mL in terms of Compound A) for each concentration was added to each tube. The reaction mixture was stirred in an incubator at 37°C for 8 hours.

(2) 400 μL of methanol was added to 100 μL of the blood sample obtained in (1). After stirring, 400 μL of chloroform was added, followed by further stirring. 120 μL of ultrapure water was added thereto, followed by stirring and then centrifugation at 4°C and 10000 × g for 15 min. 400 μL of the aqueous phase was recovered and added to an ultrafiltration tube, followed by centrifugation at 12°C and 9200 × g for 2 hours. The filtrate was dried under reduced pressure at 40°C for 2 hours by using a centrifugal evaporator. After drying, the resulting product was stored in a freezer at -80°C.

(3) The samples obtained in (2) were returned to room temperature and 40 μL of ultrapure water was added to each sample. The mixture was stirred and centrifuged at 4°C and 21500 × g for 5 min. 35 μL of the supernatant was transferred to a vial. After storage at 4°C, XMP, GMP and GTP in the samples were determined in the LC-MS/MS determination instrument and LC conditions described in Example 3 (3), and MS/MS conditions shown in Table 15.

[Table 15]

| Determining object | Determining conditions | Precursor ion (m/z) /Product ion (m/z) | Temperature (gas temperature of turbo heater)(°C) | Voltage of ion introduction unit (orifice plate) (V) |
|---|---|---|---|---|
| XMP | A | 365.0/97.0 | 600 | 50 |
| | B | 365.0/153.0 | | |
| GMP | E | 364.0/152.0 | | |
| GTP | F | 524.0/152.0 | | |

(4) Amounts of XMP, GMP and GTP (%) were calculated assuming that the XMP, GMP and GTP values of a non-Compound A treated group (peak area value) are defined as 100%. The results are shown in Table 16.

[Table 16]

| Concentration of Compound A (μg/mL) | Amount of each compound in whole blood (%) | | |
|---|---|---|---|
| | XMP | GMP | GTP |
| 0 | 100 | 100 | 100 |
| 1 | 55 | 85 | 89 |
| 10 | 30 | 85 | 86 |
| 100 | 13 | 91 | 79 |

The amount of XMP in whole blood was decreased with an increasing concentration of Compound A. Meanwhile, the amounts of GMP and GTP in whole blood exhibited no great change even when the concentration of Compound A is increased. From this result, it was shown that the therapeutic effects of Compound A cannot be evaluated in downstream metabolites of XMP, such as GMP or GTP, and the therapeutic effects of Compound A can be evaluated in XMP.

Example 9 Gradient Conditions of LC

The sample was prepared in the same manner as those described in Examples 3(1-1) and (1-2). The sample was dissolved in water, followed by centrifugation, and the supernatant was recovered to serve as a determination sample. The determination sample and XMP standard were subjected to LC-MS/MS. The configuration of an apparatus and determining conditions are given below.

(LC-MS/MS)

LC: UFLC (manufactured by Shimadzu Corporation)
MS: QTRAP 5500 (manufactured by AB SCIEX)

(LC)

Column: SeQuant ZIC-pHILIC 5 $\mu$m, polymeric PEEK 150×2.1 mm metal-free HPLC column (manufactured by Merck Ltd.)
Column oven temperature: 40°C
Mobile phase A: 50 mmol/L of ammonium bicarbonate (adjusted to pH 9.4 by 25 mass% aqueous ammonia solution)
Mobile phase B: acetonitrile
Feeding conditions: 0.3 mL/min
Injection volume: 2 $\mu$L (Condition a), 5 $\mu$L (Condition b)

The gradient cycles of Conditions a and b are shown in Tables 17 and 18. % in Tables 17 and 18 indicates volume%.

Condition a

[Table 17]

| Time (min) | 0 | 5.83 | 5.84 | 9.83 | 9.84 | 13.83 |
|---|---|---|---|---|---|---|
| Mobile phase A (%) | 30 | 65 | 100 | 100 | 30 | 30 |
| Mobile phase B (%) | 70 | 35 | 0 | 0 | 70 | 70 |

Condition b

[Table 18]

| Time (min) | 0 | 2.5 | 10 | 13 | 16 | 16.01 | 21 |
|---|---|---|---|---|---|---|---|
| Mobile phase A (%) | 20 | 35 | 35 | 100 | 100 | 20 | 20 |
| Mobile phase B (%) | 80 | 65 | 65 | 0 | 0 | 80 | 80 |

(MS/MS)

Ionization: ESI positive

Scan Mode: MRM

The determining conditions are shown in Table 19.

[Table 19]

| Determining object | Determining conditions | Precursor ion (m/z) /Product ion (m/z) | Temperature (gas temperature of turbo heater) (°C) | Voltage of ion introduction unit (orifice plate) (V) |
|---|---|---|---|---|
| XMP | A B | 365.0/97.0 365.0/153.0 | 600 | 50 |

The obtained determination results are shown in Fig. 8.

It has been confirmed that a degree of separation between XMP and contaminants in the sample is improved, and thereby XMP in the sample can be detected with higher specificity in the case of using an isocratic method in XMP elution of LC condition b, as compared with the case of using a gradient method of LC condition a. Furthermore, in LC condition b, it was possible to increase an injection volume of the sample to 2.5 times as that of LC condition a.

**Industrial Applicability**

The prediction method can predict an effective dose of Compound A or a salt thereof or a hydrate thereof for an MDS patient in a simple operation and a short time. Further, it is possible to predict whether or not an MDS patient is sensitive to a treatment using Compound A or a salt thereof or a hydrate thereof in a simple operation and a short time.

The determining method of the present invention can accurately determine amounts of XMP which is present in trace amounts in blood.

The prediction apparatus of the present invention can be used to predict an effective dose of Compound A or a salt thereof or a hydrate thereof for an MDS patient in a simple operation and a short time. Further, it can be used for predicting whether or not an MDS patient is sensitive to a treatment using Compound A or a salt thereof or a hydrate thereof in a simple operation and a short time.

Since the treatment agent and the treatment method is used in the treatment of an MDS patient who has been predicted to be sensitive to a treatment using Compound A or a salt thereof or a hydrate thereof, it is possible to avoid unnecessary administration to the patient who exhibits no sensitivity to Compound A or a salt thereof or a hydrate thereof.

**Claims**

1. A method for predicting an effective dose of 5-hydroxy-1H-imidazole-4-carboxamide or a salt thereof or a hydrate thereof for a myelodysplastic syndrome patient, comprising:

   (a) a step of determining (i) amounts of xanthosine monophosphate in whole blood collected from the patient before the administration of 5-hydroxy-1H-imidazole-4-carboxamide or a salt thereof or a hydrate thereof, and (ii) amounts of xanthosine monophosphate in the whole blood collected from the patient after the administration of 5-hydroxy-1H-imidazole-4-carboxamide or a salt thereof or a hydrate thereof, or amounts of xanthosine monophosphate in the whole blood obtained by bringing the blood collected from the patient before the administration of 5-hydroxy-1H-imidazole-4-carboxamide or a salt thereof or a hydrate thereof into contact with 5-hydroxy-1H-imidazole-4-carboxamide or a salt thereof or a hydrate thereof;
   (b) a step of determining a variation of xanthosine monophosphate from the amounts of xanthosine monophosphate obtained in step (a); and
   (c) a step of predicting an effective dose of 5-hydroxy-1H-imidazole-4-carboxamide or a salt thereof or a hydrate thereof from the variation of xanthosine monophosphate obtained in step (b),

   wherein step (a) includes the following steps:

   (a1) a step of determining xanthosine monophosphate in whole blood in two different determining conditions by mass spectrometry;

(a2) a step of determining an ionic strength ratio of xanthosine monophosphate in whole blood in two different determining conditions obtained in step (al); and

(a3) a step of checking whether the ionic strength ratio of xanthosine monophosphate in whole blood obtained in step (a2) is less than 1,

wherein the two different determining conditions are detection ions and/or ionization conditions in mass spectrometry, wherein the ionic strength ratio is calculated as follows:

$$\text{ionic strength ratio} = \text{(strength of ions under the other determining condition)}/\text{(strength of product ion of 97.0 (m/z), when 365.0 (m/z) is taken as a precursor ion)}.$$

2. The method according to claim 1, wherein step (a1) is a step of determining xanthosine monophosphate in whole blood in two different determining conditions by liquid chromatography-mass spectrometry.

3. The method according to claim 2, wherein the pH value of the mobile phase used for liquid chromatography is 7 to 11.

4. The method according to claim 2 or 3, wherein the salt concentration of the mobile phase used for liquid chromatography is 5 mmol/L to 300 mmol/L.

5. The method according to any one of claims 2 to 4, wherein the mobile phase used for liquid chromatography is a mobile phase including an aqueous ammonium bicarbonate solution.

6. The method according to any one of claims 2 to 5, wherein the liquid chromatography is hydrophilic interaction chromatography.

7. The method according to any one of claim 1 to 5 wherein step (c) is a step including

(c1) a step of expressing the relationship between the post-administration time (t) or contact time (t) of 5-hydroxy-1H-imidazole-4-carboxamide or a salt thereof or a hydrate thereof and the variation (Y) of xanthosine monophosphate obtained in step (b) in terms of a function therebetween expressed by the formula Y=f(t), and

(c2) a step of predicting an effective dose of 5-hydroxy-1H-imidazole-4-carboxamide or a salt thereof or a hydrate thereof from the function obtained in step (c1).

8. The method according to claim 7, wherein step (c2) is a step including a step of predicting that the dose of 5-hydroxy-1H-imidazole-4-carboxamide or a salt thereof or a hydrate thereof at which a variation of xanthosine monophosphate is greater than or equal to a predetermined variation in a given treatment period is an effective dose.

9. The method according to claim 7, wherein step (c2) is a step including a step of predicting that the dose of 5-hydroxy-1H-imidazole-4-carboxamide or a salt thereof or a hydrate thereof at which a variation of xanthosine monophosphate is greater than or equal to a predetermined variation of 45% or more in a period longer than or equal to a specified proportion of a given treatment period is an effective dose, which is longer than or equal to 40% of the given treatment period.

10. A method for predicting whether or not a myelodysplastic syndrome patient is sensitive to a treatment using 5-hydroxy-1H-imidazole-4-carboxamide or a salt thereof or a hydrate thereof, comprising:

(a) a step of determining (i) amounts of xanthosine monophosphate in the whole blood collected from the patient before the administration of 5-hydroxy-1H-imidazole-4-carboxamide or a salt thereof or a hydrate thereof, and (ii) amounts of xanthosine monophosphate in the whole blood collected from the patient after the administration of 5-hydroxy-1H-imidazole-4-carboxamide or a salt thereof or a hydrate thereof, or amounts of xanthosine monophosphate in the whole blood obtained by bringing the blood collected from the patient before the administration of 5-hydroxy-1H-imidazole-4-carboxamide or a salt thereof or a hydrate thereof into contact with 5-hydroxy-1H-imidazole-4-carboxamide or a salt thereof or a hydrate thereof;

(b) a step of determining a variation of xanthosine monophosphate from the amounts of xanthosine monophosphate obtained in step (a); and
(c) a step of predicting whether or not a patient is sensitive to a treatment using 5-hydroxy-1H-imidazole-4-carboxamide or a salt thereof or a hydrate thereof from the variation of xanthosine monophosphate obtained in step (b),

wherein step (a) includes the following steps:

(a1) a step of determining xanthosine monophosphate in whole blood in two different determining conditions by mass spectrometry;
(a2) a step of determining an ionic strength ratio of xanthosine monophosphate in whole blood in two different determining conditions obtained in step (al); and
(a3) a step of checking whether the ionic strength ratio of xanthosine monophosphate in whole blood obtained in step (a2) is less than 1,

wherein the two different determining conditions are detection ions and/or ionization conditions in mass spectrometry, wherein the ionic strength ratio is calculated as follows:

$$\text{ionic strength ratio} = \text{(strength of ions under the other determining condition)/(strength of product ion of } 97.0 \text{ (m/z), when } 365.0 \text{ (m/z) is taken as a precursor ion).}$$

11. The method according to claim 10, wherein step (a1) is a step of determining xanthosine monophosphate in whole blood in two different determining conditions by liquid chromatography-mass spectrometry.

12. The method according to any one of claims 10 or 11, wherein step (c) is a step including

(c1) a step of expressing the relationship between the post-administration time (t) or contact time (t) of 5-hydroxy-1H-imidazole-4-carboxamide or a salt thereof or a hydrate thereof and the variation (Y) of xanthosine monophosphate obtained in step (b) in terms of a function therebetween expressed by the formula Y=f(t), and
(c2) a step of predicting whether or not a patient is sensitive to a treatment using 5-hydroxy-1H-imidazole-4-carboxamide or a salt thereof or a hydrate thereof from the function obtained in step (c1).

13. The method according to claim 12, wherein step (c2) is a step including a step of predicting that a patent is sensitive to a treatment using 5-hydroxy-1H-imidazole-4-carboxamide or a salt thereof or a hydrate thereof, in the case where the variation of xanthosine monophosphate is greater than or equal to a predetermined variation of 45% or more in a given treatment period.

14. The method according to claim 12, wherein step (c2) is a step including a step of predicting that a patent is sensitive to a treatment using 5-hydroxy-1H-imidazole-4-carboxamide or a salt thereof or a hydrate thereof, in the case where the variation of xanthosine monophosphate is greater than or equal to a predetermined variation of 45% or more in a period longer than or equal to a specified proportion of a given treatment period, which is longer than or equal to 40% of the given treatment period.

**Patentansprüche**

1. Verfahren zum Vorhersagen einer effektiven Dosis von 5-Hydroxy-1H-imidazol-4-carboxamid oder einem Salz davon oder einem Hydrat davon für einen myelodysplastischen Syndrompatienten, enthaltend:

(a) einen Schritt der Bestimmung (i) von Mengen von Xanthosinmonophosphat im Gesamtblut, das von dem Patienten vor der Verabreichung von 5-Hydroxy-1H-imidazol-4-carboxamid oder einem Salz davon oder einem Hydrat davon gesammelt ist, und (ii) von Mengen von Xanthosinmonophosphat im Gesamtblut, das vom Patienten nach Verabreichung von 5-Hydroxy-1H-imidazol-4-carboxamid oder einem Salz davon oder einem Hydrat davon gesammelt ist, oder Mengen von Xanthosinmonophosphat im Gesamtblut, erhalten durch Kontaktieren des Blutes, das von dem Patienten gesammelt ist, vor der Verabreichung von 5-Hydroxy-1H-imidazol-4-carboxamid oder einem Salz davon oder einem Hydrat davon, mit 5-Hydroxy-1H-imidazol-4-carboxamid oder einem Salz davon oder einem Hydrat davon,

(b) einen Schritt der Bestimmung einer Variation von Xanthosinmonophosphat von den Mengen von Xanthosinmonophosphat, erhalten im Schritt (a), und

(c) einen Schritt der Vorhersage einer effektiven Menge von 5-Hydroxy-1H-imidazol-4-carboxamid oder einem Salz davon oder einem Hydrat davon von der Variation von Xanthosinmonophosphat, erhalten im Schritt (b),

worin Schritt (a) die folgenden Schritte enthält:

(a1) einen Schritt zum Bestimmen von Xanthosinmonophosphat im Gesamtblut in zwei unterschiedlichen Bestimmungsbedingungen durch Massenspektrometrie,

(a2) einen Schritt der Bestimmung eines ionischen Stärkenverhältnisses von Xanthosinmonophosphat im Gesamtblut in zwei unterschiedlichen Bestimmungsbedingungen, erhalten im Schritt (a1), und

(a3) einen Schritt der Überprüfung, ob das ionische Stärkenverhältnis von Xanthosinmonophosphat im Gesamtblut, erhalten im Schritt (a2), weniger als 1 ist,

worin die beiden unterschiedlichen Bestimmungsbedingungen Bestimmungsionen und/oder Ionisierungsbedingungen in der Massenspektrometrie sind,

worin das ionische Stärkenverhältnis berechnet wird wie folgt:

```
ionisches Stärkenverhältnis = (Stärke von Ionen unter
der anderen Bestimmungsbedingung)/(Stärke von Produktion
von 97.0 (m/z), wenn 365,0 (m/z) als Vorläuferion
verwendet wird).
```

2. Verfahren nach Anspruch 1, worin Schritt (a1) ein Schritt zur Bestimmung von Xanthosinmonophosphat im Gesamtblut in zwei unterschiedlichen Bestimmungsbedingungen durch Flüssigchromatographie-Massenspektrometrie ist.

3. Verfahren nach Anspruch 2, worin der pH-Wert der mobilen Phase, die für Flüssigchromatographie verwendet wird, 7 bis 11 ist.

4. Verfahren nach Anspruch 2 oder 3, worin die Salzkonzentration der mobilen Phase, die für die Flüssigchromatographie verwendet wird, 5 bis 300 mmol/l ist.

5. Verfahren nach einem der Ansprüche 2 bis 4, worin die mobile Phase, die für Flüssigchromatographie verwendet wird, eine mobile Phase mit einer wässrigen Ammoniumbicarbonatlösung ist.

6. Verfahren nach einem der Ansprüche 2 bis 5, worin die Flüssigchromatographie eine hydrophile Interaktionschromatographie ist.

7. Verfahren nach einem der Ansprüche 1 bis 5, worin Schritt (c) ein Schritt ist, der enthält:

(c1) einen Schritt zum Ausdrücken der Beziehung zwischen der Nachverabreichungszeit (t) oder Kontaktzeit (t) von 5-Hydroxy-1H-imidazol-4-carboxamid oder einem Salz davon oder einem Hydrat davon und der Variation (Y) von Xanthosinmonophosphat, erhalten im Schritt (b), ausgedrückt als Funktion zwischen diesen, dargestellt durch die Formel Y=f(t), und

(c2) einen Schritt der Vorhersage einer effektiven Dosis von 5-Hydroxy-1H-imidazol-4-carboxamid oder einem Salz davon oder einem Hydrat davon von der Funktion, erhalten im Schritt (c1).

8. Verfahren nach Anspruch 7, worin Schritt (c2) ein Schritt ist, enthaltend einen Schritt der Vorhersage, dass die Dosis von 5-Hydroxy-1H-imidazol-4-carboxamid oder einem Salz davon oder einem Hydrat davon, bei der eine Variation von Xanthosinmonophosphat größer oder gleich einer bestimmten Variation in einer gegebenen Behandlungsperiode ist, eine effektive Dosis ist.

9. Verfahren nach Anspruch 7, worin Schritt (c2) ein Schritt ist, enthaltend einen Schritt der Vorhersage, dass die Dosis von 5-Hydroxy-1H-imidazol-4-carboxamid oder einem Salz davon oder einem Hydrat davon, bei der eine Variation von Xanthosinmonophosphat größer oder gleich einer bestimmten Variation von 45% oder mehr in einer Periode ist, die länger oder gleich einem spezifischen Anteil einer gegebenen Behandlungsperiode ist, eine effektive Dosis ist, die länger oder gleich 40% der gegebenen Behandlungsperiode ist.

10. Verfahren zum Vorhersagen, ob ein myelodisplastischer Syndrompatient sensitiv für eine Behandlung unter Verwendung von 5-Hydroxy-1H-imidazol-4-carboxamid oder einem Salz oder einem Hydrat davon ist, enthaltend:

(a) einen Schritt der Bestimmung (i) von Mengen von Xanthosinmonophosphat im Gesamtblut, das von dem Patienten vor der Verabreichung von 5-Hydroxy-1H-imidazol-4-carboxamid oder einem Salz davon oder einem Hydrat davon gesammelt ist, und (ii) von Mengen von Xanthosinmonophosphat im Gesamtblut, das vom Patienten nach Verabreichung von 5-Hydroxy-1H-imidazol-4-carboxamid oder einem Salz davon oder einem Hydrat davon gesammelt ist, oder Mengen von Xanthosinmonophosphat im Gesamtblut, erhalten durch Kontaktieren des Blutes, das von dem Patienten gesammelt ist, vor der Verabreichung von 5-Hydroxy-1H-imidazol-4-carboxamid oder einem Salz davon oder einem Hydrat davon, mit 5-Hydroxy-1H-imidazol-4-carboxamid oder einem Salz davon oder einem Hydrat davon,
(b) einen Schritt der Bestimmung einer Variation von Xanthosinmonophosphate von den Mengen von Xanthosinmonophosphat, erhalten im Schritt (a), und
(c) einen Schritt der Vorhersage einer effektiven Menge von 5-Hydroxy-1H-imidazol-4-carboxamid oder einem Salz davon oder einem Hydrat davon von der Variation von Xanthosinmonophosphat, erhalten im Schritt (b),

worin Schritt (a) die folgenden Schritte enthält:

(a1) einen Schritt zum Bestimmen von Xanthosinmonophosphat im Gesamtblut in zwei unterschiedlichen Bestimmungsbedingungen durch Massenspektrometrie,
(a2) einen Schritt der Bestimmung eines ionischen Stärkenverhältnisses von Xanthosinmonophosphat im Gesamtblut in zwei unterschiedlichen Bestimmungsbedingungen, erhalten im Schritt (a1), und
(a3) einen Schritt der Überprüfung, ob das ionische Stärkenverhältnis von Xanthosinmonophosphat im Gesamtblut, erhalten im Schritt (a2), weniger als 1 ist,

worin die beiden unterschiedlichen Bestimmungsbedingungen Bestimmungsionen und/oder Ionisierungsbedingungen in der Massenspektrometrie sind,
worin das ionische Stärkenverhältnis berechnet wird wie folgt:

```
ionisches Stärkenverhältnis = (Stärke von Ionen unter
der anderen Bestimmungsbedingung)/(Stärke von Produktion
von 97.0 (m/z), wenn 365,0 (m/z) als Vorläuferion
verwendet wird).
```

11. Verfahren nach Anspruch 10, worin Schritt (a1) ein Schritt zur Bestimmung von Xanthosinmonophosphat im Gesamtblut in zwei unterschiedlichen Bestimmungsbedingungen durch Flüssigchromatographie-Massenspektrometrie ist.

12. Verfahren nach einem der Ansprüche 10 oder 11, worin Schritt (c) ein Schritt ist, der enthält:

(c1) einen Schritt zum Ausdrücken der Beziehung zwischen der Nachverabreichungszeit (t) oder Kontaktzeit (t) von 5-Hydroxy-1H-imidazol-4-carboxamid oder einem Salz davon oder einem Hydrat davon und der Variation (Y) von Xanthosinmonophosphat, erhalten im Schritt (b), ausgedrückt als Funktion zwischen diesen, dargestellt durch die Formel Y=f(t), und
(c2) einen Schritt der Vorhersage einer effektiven Dosis von 5-Hydroxy-1H-imidazol-4-carboxamid oder einem Salz davon oder einem Hydrat davon von der Funktion, erhalten im Schritt (c1).

13. Verfahren nach Anspruch 12, worin Schritt (c2) ein Schritt ist, umfassend einen Schritt der Vorhersage, dass ein Patient für eine Behandlung unter Verwendung von 5-Hydroxy-1H-imidazol-4-carboxamid oder einem Salz davon oder einem Hydrat davon sensibel ist in dem Fall, wenn die Variation von Xanthosinmonophosphat größer als oder gleich einer bestimmten Variation von 45% oder mehr in einer gegebenen Behandlungsperiode ist.

14. Verfahren nach Anspruch 12, worin Schritt (c2) ein Schritt ist, enthaltend einen Schritt der Vorhersage, dass ein Patient sensibel für die Behandlung unter Verwendung von 5-Hydroxy-1H-imidazol-4-carboxamid oder einem Salz davon oder einem Hydrat davon ist, wenn die Variation von Xanthosinmonophosphat größer oder gleich einer bestimmten Variation von 45% oder mehr in einer Periode ist, die länger oder gleich einem spezifischen Anteil einer gegebenen Behandlungsperiode ist, die länger oder gleich 40% der gegebenen Behandlungsperiode ist.

**Revendications**

1. Procédé de prédiction d'une dose efficace de 5-hydroxy-1H-imidazole-4-carboxamide ou d'un sel de celui-ci ou d'un hydrate de celui-ci pour un patient atteint du syndrome myélodysplasique, comprenant :

   (a) une étape de détermination (i) de quantités de monophosphate de xanthosine dans du sang total prélevé du patient avant l'administration de 5-hydroxy-1H-imidazole-4-carboxamide ou d'un sel de celui-ci ou d'un hydrate de celui-ci, et (ii) de quantités de monophosphate de xanthosine dans le sang total prélevé du patient après l'administration de 5-hydroxy-1H-imidazole-4-carboxamide ou d'un sel de celui-ci ou d'un hydrate de celui-ci, ou de quantités de monophosphate de xanthosine dans le sang total obtenu en mettant le sang prélevé du patient avant l'administration de 5-hydroxy-1H-imidazole-4-carboxamide ou d'un sel de celui-ci ou d'un hydrate de celui-ci en contact avec le 5-hydroxy-1H-imidazole-4-carboxamide ou un sel de celui-ci ou un hydrate de celui-ci ;
   (b) une étape de détermination d'une variation de monophosphate de xanthosine à partir des quantités de monophosphate de xanthosine obtenues à l'étape (a) ; et
   (c) une étape de prédiction d'une dose efficace de 5-hydroxy-1H-imidazole-4-carboxamide ou d'un sel de celui-ci ou d'un hydrate de celui-ci à partir de la variation de monophosphate de xanthosine obtenue à l'étape (b),

   dans lequel l'étape (a) inclut les étapes suivantes :

   (a1) une étape de détermination de monophosphate de xanthosine dans le sang total dans deux conditions de détermination différentes par spectrométrie de masse ;
   (a2) une étape de détermination d'un rapport d'intensité ionique du monophosphate de xanthosine dans le sang total dans deux conditions de détermination différentes obtenu à l'étape (a1) ; et
   (a3) une étape de vérification que le rapport d'intensité ionique du monophosphate de xanthosine dans le sang total obtenu à l'étape (a2) est inférieur à 1,

   dans lequel les deux conditions de détermination différentes sont des ions de détection et/ou des conditions d'ionisation en spectrométrie de masse,
   dans lequel le rapport d'intensité ionique est calculé de la manière suivante :

   $$\text{rapport d'intensité ionique} = (\text{intensité d'ions dans l'autre condition de détermination})/(\text{intensité d'ion produit de 97,0 (m/z), lorsque 365,0 (m/z) est pris en tant qu'ion précurseur}).$$

2. Procédé selon la revendication 1, dans lequel l'étape (a1) est une étape de détermination de monophosphate de xanthosine dans le sang total dans deux conditions de détermination différentes par chromatographie en phase liquide-spectrométrie de masse.

3. Procédé selon la revendication 2, dans lequel la valeur de pH de la phase mobile utilisée pour la chromatographie en phase liquide est de 7 à 11.

4. Procédé selon la revendication 2 ou 3, dans lequel la concentration en sel de la phase mobile utilisée pour la chromatographie en phase liquide est de 5 mmol/l à 300 mmol/l.

5. Procédé selon l'une quelconque des revendications 2 à 4, dans lequel la phase mobile utilisée pour la chromatographie en phase liquide est une phase mobile incluant une solution aqueuse de bicarbonate d'ammonium.

6. Procédé selon l'une quelconque des revendications 2 à 5, dans lequel la chromatographie en phase liquide est une chromatographie d'interaction hydrophile.

7. Procédé selon l'une quelconque des revendications 1 à 5 dans lequel l'étape (c) est une étape incluant

(c1) une étape d'expression de la relation entre le temps post-administration (t) ou le temps de contact (t) de 5-hydroxy-1H-imidazole-4-carboxamide ou d'un sel de celui-ci ou d'un hydrate de celui-ci et la variation (Y) de monophosphate de xanthosine obtenu à l'étape (b) en termes d'une fonction entre ceux-ci exprimée par la formule Y = f(t), et
(c2) une étape de prédiction d'une dose efficace de 5-hydroxy-1H-imidazole-4-carboxamide ou d'un sel de celui-ci ou d'un hydrate de celui-ci à partir de la fonction obtenue à l'étape (c1).

8. Procédé selon la revendication 7, dans lequel l'étape (c2) est une étape incluant une étape de prédiction que la dose de 5-hydroxy-1H-imidazole-4-carboxamide ou d'un sel de celui-ci ou d'un hydrate de celui-ci à laquelle une variation de monophosphate de xanthosine est supérieure ou égale à une variation prédéterminée dans une période de traitement donnée est une dose efficace.

9. Procédé selon la revendication 7, dans lequel l'étape (c2) est une étape incluant une étape de prédiction que la dose de 5-hydroxy-1H-imidazole-4-carboxamide ou d'un sel de celui-ci ou d'un hydrate de celui-ci à laquelle une variation de monophosphate de xanthosine est supérieure ou égale à une variation prédéterminée de 45 % ou plus dans une période plus longue que ou égale à une proportion spécifiée d'une période de traitement donnée est une dose efficace, qui est plus longue que ou égale à 40 % de la période de traitement donnée.

10. Procédé de prédiction de la sensibilité ou non d'un patient atteint du syndrome myélodysplasique à un traitement utilisant le 5-hydroxy-1H-imidazole-4-carboxamide ou un sel de celui-ci ou un hydrate de celui-ci, comprenant :

(a) une étape de détermination (i) de quantités de monophosphate de xanthosine dans le sang total prélevé du patient avant l'administration de 5-hydroxy-1H-imidazole-4-carboxamide ou d'un sel de celui-ci ou d'un hydrate de celui-ci, et (ii) de quantités de monophosphate de xanthosine dans le sang total prélevé du patient après l'administration de 5-hydroxy-1H-imidazole-4-carboxamide ou d'un sel de celui-ci ou d'un hydrate de celui-ci, ou de quantités de monophosphate de xanthosine dans le sang total obtenu par mise en contact du sang prélevé du patient avant l'administration de 5-hydroxy-1H-imidazole-4-carboxamide ou d'un sel de celui-ci ou d'un hydrate de celui-ci avec le 5-hydroxy-1H-imidazole-4-carboxamide ou un sel de celui-ci ou un hydrate de celui-ci ;
(b) une étape de détermination d'une variation de monophosphate de xanthosine à partir des quantités de monophosphate de xanthosine obtenues à l'étape (a) ; et
(c) une étape de prédiction de la sensibilité ou non d'un patient à un traitement utilisant le 5-hydroxy-1H-imidazole-4-carboxamide ou un sel de celui-ci ou un hydrate de celui-ci à partir de la variation de monophosphate de xanthosine obtenue à l'étape (b),

dans lequel l'étape (a) inclut les étapes suivantes :

(a1) une étape de détermination de monophosphate de xanthosine dans le sang total dans deux conditions de détermination différentes par spectrométrie de masse ;
(a2) une étape de détermination d'un rapport d'intensité ionique du monophosphate de xanthosine dans le sang total dans deux conditions de détermination différentes obtenu à l'étape (a1) ; et
(a3) une étape de vérification que le rapport d'intensité ionique du monophosphate de xanthosine obtenu à l'étape (a2) est inférieur à 1,

dans lequel les deux conditions de détermination différentes sont des ions de détection et/ou de conditions d'ionisation en spectrométrie de masse,
dans lequel le rapport d'intensité ionique est calculé de la manière suivante :

rapport d'intensité ionique = (intensité d'ions dans l'autre condition de détermination)/(intensité d'ion produit de 97,0 (m/z), lorsque 365,0 (m/z) est pris comme ion précurseur).

11. Procédé selon la revendication 10, dans lequel l'étape (a1) est une étape de détermination de monophosphate de xanthosine dans le sang total dans deux conditions de détermination par chromatographie en phase liquide-spectrométrie de masse.

12. Procédé selon l'une quelconque des revendications 10 ou 11, dans lequel l'étape (c) est une étape incluant

(c1) une étape d'expression de la relation entre le temps post-administration (t) ou le temps de contact (t) de 5-hydroxy-1H-imidazole-4-carboxamide ou d'un sel de celui-ci ou d'un hydrate de celui-ci et la variation (Y) de monophosphate de xanthosine obtenue à l'étape (b) en termes d'une fonction entre ceux-ci exprimée par la formule Y = f(t), et
(c2) une étape de prédiction de la sensibilité ou non d'un patient à un traitement utilisant le 5-hydroxy-1H-imidazole-4-carboxamide ou un sel de celui-ci ou un hydrate de celui-ci à partir de la fonction obtenue à l'étape (c1).

13. Procédé selon la revendication 12, dans lequel l'étape (c2) est une étape incluant une étape de prédiction de la sensibilité d'un patient à un traitement utilisant le 5-hydroxy-1H-imidazole-4-carboxamide ou un sel de celui-ci ou un hydrate de celui-ci, dans le cas où la variation de monophosphate de xanthosine est supérieure ou égale à une variation prédéterminée de 45 % ou plus dans une période de traitement donnée.

14. Procédé selon a revendication 12, dans lequel l'étape (c2) est une étape incluant une étape de prédiction de la sensibilité d'un patient à un traitement utilisant le 5-hydroxy-1H-imidazole-4-carboxamide ou un sel de celui-ci ou un hydrate de celui-ci, dans le cas où la variation de monophosphate de xanthosine est supérieure ou égale à une variation prédéterminée de 45 % ou plus dans une période plus longue que ou égale à une proportion spécifiée d'une période de traitement donnée, qui est plus longue que ou égale à 40 % de la période de traitement donnée.

## FIG. 1

## FIG. 2

## FIG. 3

## FIG. 4

# FIG. 5

DETERMINING CONDITION A · DETERMINING CONDITION B

XMP STANDARD

SAMPLE

# FIG. 6

DETERMINING CONDITION C · DETERMINING CONDITION D

XMP STANDARD

SAMPLE

# FIG. 7

DETERMINING CONDITION A'  DETERMINING CONDITION B'

XMP STANDARD

SAMPLE

# FIG. 8

CONDITION a  CONDITION b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4753935 A
- JP 53032124 A
- JP S5332124 A
- JP 58024569 A
- JP S5824569 A
- WO 2009035168 A
- WO 2013047758 A

**Non-patent literature cited in the description**

- *Cancer and Chemotherapy,* 1989, vol. 16 (1), 123-130
- *Analytical Chemistry,* 2012, vol. 84, 216-223
- *Analytical Chemistry,* 2012, vol. 84, 1994-2001
- **STRYER.** Biochemistry. 2013, 689-693